(19)

Europäisches
Patentamt
European
Patent Office
Office européen
des brevets

(11) **EP 4 234 720 A1**

(12) **EUROPEAN PATENT APPLICATION**

(43) Date of publication:
**30.08.2023   Bulletin 2023/35**

(21) Application number: **22382165.3**

(22) Date of filing: **25.02.2022**

(51) International Patent Classification (IPC):
***C12Q 1/6886*** (2018.01)

(52) Cooperative Patent Classification (CPC):
**C12Q 1/6886;** C12Q 2600/118; C12Q 2600/154

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
 • **Consejo Superior de Investigaciones Cientificas
   28006 Madrid (ES)**
 • **Fundacion Para La Investigacion e Innovacion
   Biosanitaria Principado de Asturias
   33011 Oviedo (ES)**

(72) Inventors:
 • **Rodríguez-Rodero, Sandra
   28006 Madrid (ES)**
 • **Tejedor Vaquero, Juan Ramón
   28006 Madrid (ES)**

 • **Fernández Fernández, Agustín
   28006 Madrid (ES)**
 • **Fernández Fraga, Mario
   28006 Madrid (ES)**
 • **Delgado Álvarez, Elías
   33011 Oviedo (ASTURIAS) (ES)**
 • **Morales Sánchez, Paula
   33011 Oviedo (ASTURIAS) (ES)**
 • **Menendez Torre, Edelmiro
   33011 Oviedo (ASTURIAS) (ES)**

(74) Representative: **Pons
Glorieta Rubén Darío 4
28010 Madrid (ES)**

Remarks:
The complete document including Reference
Table(s) and the Sequence Listing(s) can be
downloaded from the EPO website

(54) **EPIGENETIC BIOMARKERS FOR THE DIAGNOSIS OF THYROID CANCER**

(57)   The invention relates to an *in vitro* method for the diagnosis and/or prognosis of thyroid carcinoma, discriminating between benign and malignant nodules, by comparing the methylation level of the cytosine residue of various CpG sites, particularly the CpG sites at position 97,858,492-97,858,493 of the human chromosome 13, position 74,957,673-74,957,674 of the human chromosome 14 and/or position 5,672,830-5,672,831 of the human chromosome 20. Further, the present invention relates to the use of the methylation levels of said CpG sites and the kit for *in vitro* determination of the methylation levels.

**EP 4 234 720 A1**

**Description**

The invention relates to an *in vitro* method for the diagnosis and/or prognosis of thyroid carcinoma, particularly, to a method for *in vitro* discriminating between benign and malignant nodules by determining the methylation level of one or various CpG sites within human genome.

**BACKGROUND ART**

Thyroid nodules are commonly seen in clinical practice. The use of ultrasonography-guided fine needle aspiration cytology (FNAC) can detect nodules in 25-50% of symptomatic adults, who are mainly women, and this incidence increases with age. Findings based on FNAC are the mainstay of clinical decisions, with most thyroid nodules being diagnosed as benign (62 to 85%), thus removing the need for diagnostic surgery, or as malignant. But 15-30%, are classified as indeterminate, which includes those that are atypia of undetermined significance or a follicular lesion of undetermined significance (AUS/FLUS; Bethesda III), follicular neoplasm or suspected follicular neoplasm (FN/SFN; Bethesda IV). The estimated risk of malignancy is 5-15% in class III and 15-30% in class IV tumours and most patients with these indeterminate nodules are routinely referred for diagnostic thyroid surgery. However, most of these indeterminate nodules (65-85% of them) are classified as benign after the final histologic evaluation. For these patients, surgery is (in hindsight) unnecessary and puts them at risk of surgical complications, permanent hypocalcemia, voice change and lifelong exogenous levothyroxine therapy.

Genetic alterations related to thyroid cancer have been used as molecular markers to distinguish benign and malignant lesions. There are currently at least three commercial methods that assess the degree of malignancy of a thyroid nodule: ThyroSeq v3 (*CBLPath, Rye Brook, NY, USA*) interrogates the genetic status of a given sample using a panel of 112 recurrent alterations associated with thyroid carcinomas. This method is reported to have high sensitivity (94%) and specificity (82%) for thyroid FNACs classified as AUS/FLUS or FN/SFN (Nikiforova MN., et al 2018, Analytical performance of the ThyroSeq v3 genomic classifier for cancer diagnosis in thyroid nodules. Cancer. John Wiley and Sons Inc.; 124:1682-90*)*. On the other hand, the Afirma gene expression classifier (GEC) *(Veracyte, San Francisco, CA, USA)* analyses the expression pattern of mRNA extracted from FNAC and classifies the samples as normal or suspicious based on the analysis of 167 differentially expressed genes identified in benign and malignant thyroid nodules *(*Alexander EK., et al 2012, Preoperative Diagnosis of Benign Thyroid Nodules with Indeterminate Cytology. New England Journal of Medicine; 367:705-15*)*. In this context, the PPV of a suspicious nodule was found to range from 30.1 to 39.3% with a NPV of 94-95%. The last method, ThyraMIR and ThyGenX combination testing (*Interpace Diagnostics, Parsippany, NJ, USA*), is comprised of the analysis of 10 microRNA-based expression classifier linked to a next-generation sequencing (NGS) platform to identify more than 100 genetic alterations across 8 genes associated with thyroid malignancy in FNAC samples. This method has demonstrated a sensitivity and specificity of 89 and 85%, respectively, with NPV of 94% and PPV of 74% *(*Labourier E., et al 2015, Molecular testing for miRNA, mRNA, and DNA on fine-needle aspiration improves the preoperative diagnosis of thyroid nodules with indeterminate cytology. Journal of Clinical Endocrinology and Metabolism. Endocrine Society; 100:2743-50*)*.

Despite the tangible improvement in thyroid cancer diagnosis offered by these technologies, multiple studies have found significant differences in the diagnostic potential of these molecular methods, especially in indeterminate nodules. Given the economic impact of these approaches on national health care systems, and the difficulties around specimen sampling and preservation, which is critical to ensure an accurate molecular testing result, there is a critical need to develop novel cost-effective methods to improve preoperative diagnostic evaluation for patients with indeterminate FNACs.

DNA methylation is one of the main epigenetic modifications and its role in the initiation, development, and metastasis of human cancers has been widely described. For example, WO2008097543 A2 discloses DNA biomarker sequences that are differentially methylated in samples from normal individuals and individuals with cancer and a method for determining the presence or absence of cancer in an individual comprising determining the methylation status of at least one cytosine within a DNA region in a sample from an individual where the DNA region is a sequence selected from the group consisting of a number SEQ IDs.

While several studies have demonstrated the role of aberrant DNA methylation in thyroid tumorigenesis *(*Zafon C., et al 2019, DNA methylation in thyroid cancer. Endocrine-Related Cancer. BioScientifica Ltd.; page R415-39*)*, most of them have focused on well-differentiated papillary thyroid carcinoma (PTC), which accounts for 85% of thyroid cancer or follicular thyroid carcinoma (FTC) (about 10% of thyroid cancers). WO2016020551 A1 discloses a method of distinguishing a thyroid cancer type or risk thereof, comprising the step of determining the DNA methylation status of thyroid cancer genes of a sample of a subject, wherein the thyroid cancer genes are selected from a list with about 63 genes, and comparing the methylation status of said genes with a control sample, thereby identifying thyroid cancer DNA in the sample.

US 2017/0022570 A1 discloses a method of determining benign nodules from thyroid cancer in a subject that is found to have a thyroid nodule, wherein said method comprising detecting methylation or unmethylation of a thyroid nodule DNA molecule (plurality of specific methylation sites) of said subject. Particularly, this document suggests that DNA methylation analysis of 258 DNA regions in thyroid specimens can serve as a potential diagnostic tool for the determination of malignancy.

However, it is still necessary the provision of alternative low-cost methods with high sensitivity and specificity for the diagnosis and prognosis of thyroid cancer and for the discrimination between benign and malignant nodules in difficult-to-diagnose cases, particularly with a reduced number of methylation probes.

**DESCRIPTION OF THE INVENTION**

The inventors provide an alternative *in vitro* method for discriminating between benign and malignant nodules in difficult-to-diagnose thyroid cancers by determining the methylation level of various CpG sites, particularly the differentially methylated CpG (dmCpG) sites at position 97,858,492-97,858,493 of the human chromosome 13 (cg17154646, GRCh37/hg19 coordinate system), position 74,957,673-74,957,674 of the human chromosome 14 (cg21915100, GRCh37/hg19 coordinate system) and/or position 5,672,830-5,672,831 of the human chromosome 20 (cg10189462, GRCh37/hg19 coordinate system).

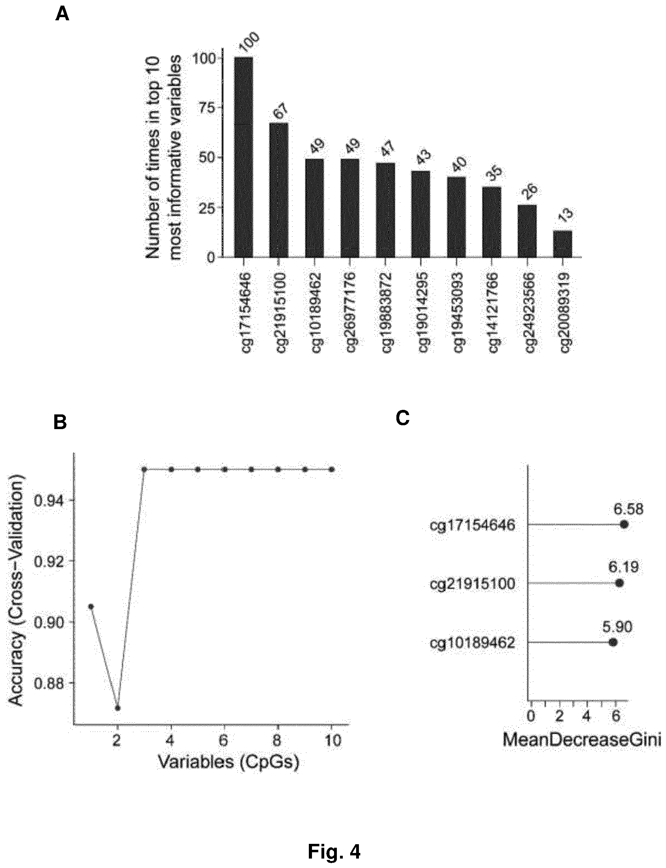

The inventors used 12,845 dmCpG sites, obtained in restrictive filtering approach, to build a robust Random Forest classification model based on prognostic features for the discrimination of benign and malignant thyroid lesions with a training dataset composed of benign (nodular hyperplasia or NH) and malignant (follicular thyroid carcinoma or FTC and follicular variant of papillary thyroid carcinoma or FVPTC) samples. By selecting the most informative and robust dmCpGs, a minimal classification system was obtained constituted by three CpG probes (cg17154646, cg21915100 and cg10189462) located, respectively, in the vicinities of the MBNL2, NPC2 and GPCPD1 genes (Figure 4).

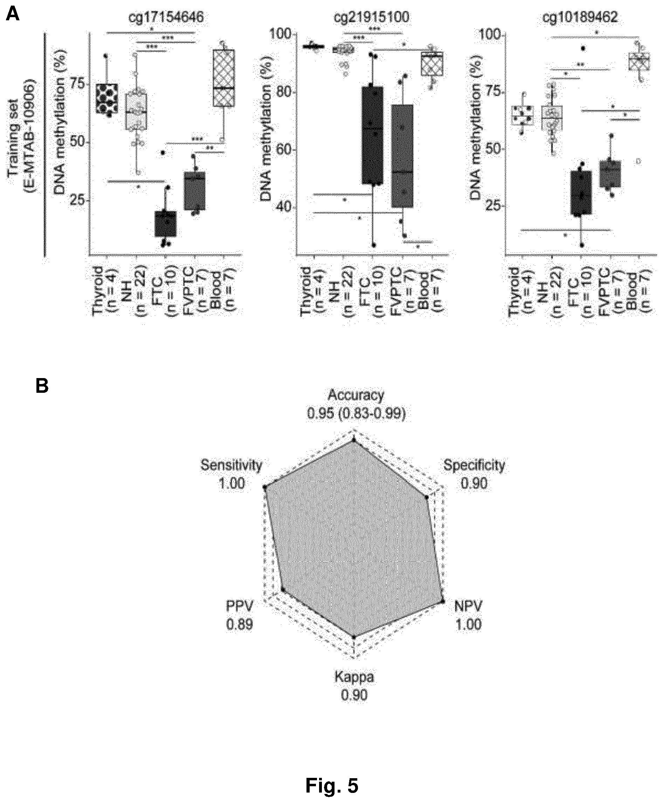

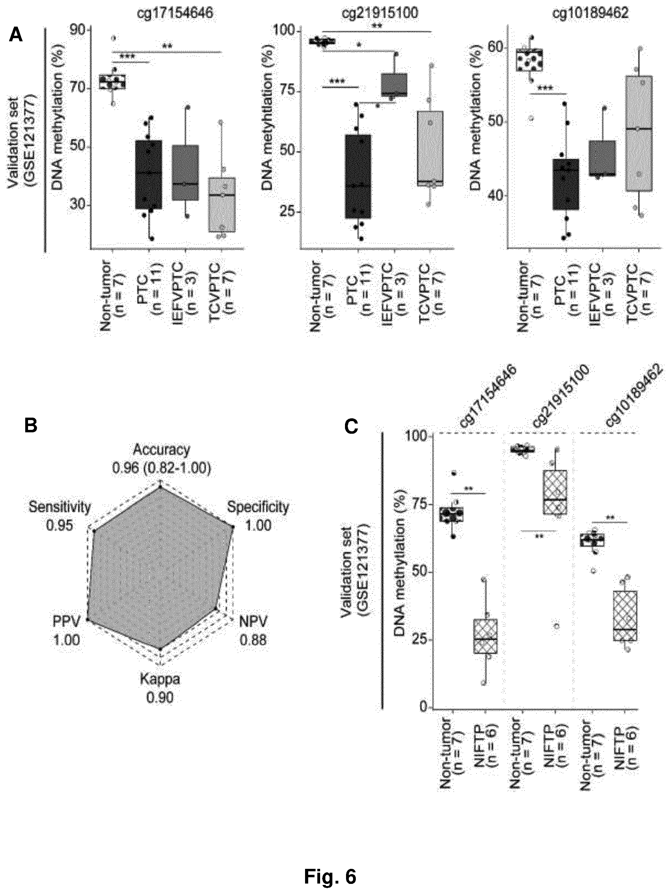

The significant discrimination of benign from malignant samples in the training dataset was observed at the DNA methylation level using any one of the individual CpG sites. Further, when all three of these CpGs were simultaneously implemented in the Random Forest classifier using a cross-validation approach, an accuracy of 0.95 with a sensitivity of 1.00 (17 of 17 malignant samples) and a specificity of 0.90 (20 out of 22 benign samples) in the training set were obtained (Figure 5). The inventors subsequently validated the minimal classification system, and this three-CpG classification system was able to discriminate between the benign (non-tumour) and the malignant thyroid carcinoma subtypes (Figure 6), indicating that the DNA methylation status of these samples can be also monitored by a less expensive and faster experimental approach and confirming the discrimination power of the minimal epigenetic classification system.

Thus, a first aspect of the present invention related to an *in vitro* method for the diagnosis and/or prognosis of thyroid carcinoma, hereinafter "the method of the invention", comprising:

(i) determining, in a biological sample isolated from a human, the methylation level of a cytosine residue of at least one CpG site, wherein the CpG site is located at position 97,858,492-97,858,493 of the human chromosome 13, position 74,957,673-74,957,674 of the human chromosome 14 and/or position 5,672,830-5,672,831 of the human chromosome 20, and

(ii) comparing the methylation level of the CpG site obtained in (i) to a threshold value, wherein a methylation level below the threshold value is indicative of presence of thyroid carcinoma in the human and the prognosis is negative; and/or

applying a machine learning algorithm, comprising the steps of:

(a) determining, in a training dataset, the methylation level or value of the cytosine residues of 3 CpG sites located at position 97,858,492-97,858,493 of the human chromosome 13, position 74,957,673-74,957,674 of the human chromosome 14 and position 5,672,830-5,672,831 of the human chromosome 20;

(b) generating a random forest model, which combines the methylation levels or values of the cytosine residues of the 3 CpG sites from the training dataset, by generating of a total of 5000 trees per model with a cut-off value for a benign set of 0.7 of the total votes;

(c) determining a prediction score of the biological sample isolated, being the prediction score a percentage of benign and malign votes predicted by the random forest model in each of the tree, by introducing the methylation level of the cytosine residues of the 3 CpG sites determined, and;

(d) comparing the prediction score obtained in (c) with a threshold value, wherein a prediction score above the threshold value is indicative of presence of thyroid carcinoma (malignant thyroid carcinoma) in the human.

In a particular embodiment, the method of the invention comprises the step (i) and (ii), preferably the step (a) to (d).

As used herein, the term "diagnosing" refers to the identification of the nature of an illness, disease or other problem by examination of the symptoms, parameters or biomarkers in a subject, particularly the discrimination of benign (non-tumour) and malignant thyroid (or thyroid carcinoma) samples. In the context of the present invention, the parameter required for diagnosis is methylation levels of at least one cytosine residue of a CpG site in a biological sample of the subject.

As used herein, the term "prognosis" refers to the process of predicting the likely or expected development of a disease (in the present case thyroid carcinoma) or determining the response of a subject to a therapy, including whether the signs and symptoms will improve or worsen (and how quickly) or remain stable over time. The term "prediction" is used herein to refer to the likelihood that a patient will have a particular clinical outcome. The clinical outcome may be positive or negative. The method of the present invention can also be used clinically to make treatment decisions by choosing the most appropriate treatment modalities for any particular patient. The predictive method of the present invention is a valuable tool in predicting if a patient is likely to respond favorably to a treatment regimen. In the context of the present invention, the expression "the prognosis of the subject is negative" means that the subject presents a thyroid tumor or thyroid carcinoma (malignant tumor), that is, the subject has thyroid cancer. On the contrary, the expression "the prognosis of the subject is positive" means that the subject does not present a thyroid tumor or thyroid carcinoma, that is, the subject has benign nodule (non-tumour). As used herein, the terms "cancer", "carcinoma" and "tumor" are considered equivalents. In the present invention "clinical outcome" is understood as the expected course of a disease. It denotes the doctor's prediction of how a subject's disease will progress, and whether there is chance of recovery o recurrence.

Firstly, the method of the invention comprises determining the methylation level or value of a cytosine residue of at least one CpG site in a biological sample isolated from a human.

As used herein, the term "methylation" refers to the presence of a methyl group added (for example, by the action of a DNA methyl transferase enzyme) to a cytosine base or bases in a region of nucleic acid, e.g., genomic DNA, particularly cytosine methylation at positions C5 or N4 of cytosine.

As used herein, the terms "methylation level", "methylation value" or "methylation percentage" refers to the average methylation of the cytosine residue in a CpG site, based on the fraction of cells in a biological sample, which do or do not have a methylation group on such cytosines, in terms of percentage (%), represented as 0 (or 0%) if all the residues of the cell population are unmethylated and 1 (or 100%) when the residues of the population are totally methylated, considering the existence of different methylation gradients between these intervals . Methylation level may also alternatively describe whether a single CpG di-nucleotide is methylated.

As used herein, the term "CpG" or "CG" site refers to site or regions in a polynucleotide DNA sequence where a cytosine nucleotide is followed by a guanine nucleotide located sequentially in the linear sequence of bases along its 5'→3' direction, shorthand for 5'-C-phosphate-G-3', that is, cytosine and guanine separated by only one phosphate group. The CpG notation is used to distinguish this single-stranded linear sequence from the CG base-pairing of cytosine and guanine for double-stranded sequences.

Particularly, the method of the invention refers to determinate the methylation level of a cytosine residue of at least one CpG site in a biological sample isolated from a human, wherein the CpG site is at position 97,858,492-97,858,493 of the human chromosome 13 (cg17154646), position 74,957,673-74,957,674 of the human chromosome 14 (cg21915100) and/or position 5,672,830-5,672,831 of the human chromosome 20 (cg10189462), hereinafter the "CpG sites of the invention", preferably the CpG site is at position 97,858,492-97,858,493 of the human chromosome 13 (cg17154646).

The CpG site named as cg17154646 is at position 97,858,492-97,858,493 of the human chromosome 13 (chr13:97,858,492-97,858,493). The genomic position is calculated based on the version GRCh37 (release 105) of the Human Genome, as annotated by the official public site of access to the genome Ensembl and accessed in December 2021.

The CpG site named as cg21915100 is at position 74,957,673-74,957,674 of the human chromosome 14 (chr14:74,957,673-74,957,674). The genomic position is calculated based on the version GRCh37 (release 105) of the Human Genome, as annotated by the official public site of access to the genome Ensembl and accessed in December 2021.

The CpG site named as cg10189462 is at position 5,672,830-5,672,831 of the human chromosome 20 (chr20:5,672,830-5,672,831). The genomic position is calculated based on the version GRCh37 (release 105) of the Human Genome, as annotated by the official public site of access to the genome Ensembl and accessed in December 2021.

As the skilled person in the art knows, a double-stranded DNA molecule will be composed of two strands with sequences that are reverse complements of each other. Two strands are differentiated as the "sense" or "positive (+)" strand and the "antisense" or "negative (-) strand. An individual strand of DNA is referred to as positive-sense or simply sense, if its nucleotide sequence corresponds directly to the sequence of an RNA transcript which is translated or translatable into a sequence of amino acids. The negative strand of the double-stranded DNA molecule is referred to reverse complementary to both the positive-sense strand and the RNA transcript.

Thus, in the method of the invention the methylated cytosine residue, in a CpG site, can be located on either positive (+) or negative (-) strand. Particularly, other preferred embodiment of the method of the invention refers to determinate the methylation level of a cytosine residue of at least one CpG site in a biological sample isolated from a human, wherein methylated cytosine residue is at position 97,858,492 of the positive strand (+) of the human chromosome 13, position 74,957,674 of the negative strand (-) of the human chromosome 14 and/or position 5,672,830 of the positive strand (+) of the human chromosome 20, preferably the methylated cytosine residue is at position 97,858,492 of the positive strand (+) of the human chromosome 13 .

The significant discrimination of benign from malignant samples in the training dataset was observed at the DNA methylation level using any one of the individual CpG sites. However, when all three of these CpG sites were simultaneously implemented in the Random Forest classifier, a sensitivity and specificity improved were observed.

Thus, in other particular embodiment the method of the invention comprises determining the methylation level of a cytosine residue of the CpG sites located at position 97,858,492-97,858,493 of the human chromosome 13 (cg17154646), position 74,957,673-74,957,674 of the human chromosome 14 (cg21915100) and/or position 5,672,830-5,672,831 of the human chromosome 20 (cg10189462).

In other more particular embodiment, the method of the invention comprises determining the methylation levels of the cytosine residue of the CpG sites located at position 97,858,492-97,858,493 of the human chromosome 13 (cg17154646), position 74,957,673-74,957,674 of the human chromosome 14 (cg21915100) and position 5,672,830-5,672,831 of the human chromosome 20 (cg10189462).

As defined above, the method of the invention comprises determining the methylation level of a cytosine residue of the CpG site in a biological sample isolated from a human.

Any method for determining or detecting methylation levels can be used in the methods of the present invention. Examples of methods for determining methylation levels at the CpG sites include, but are not limited to, bisulfite pyrosequencing, methylation-sensitive single nucleotide primer extension (MS-SnuPE), methylation-specific PCR (MSP), Sanger bisulfite sequencing, Bisulfite Restriction Site Associated DNA (BisRAD), methylation-sensitive restriction enzyme-based methods, microarray-based methods, whole-genome bisulfite sequencing (WGBS, MethylC-seq or BS-seq), reduced-representation bisulfite sequencing (RRBS), and/or enrichment-based methods such as MeDIP-seq, MBD-seq, or MRE-seq when combined with bisulfite conversion. Kits for the above methods can include, e.g., one or more of methylation-dependent restriction enzymes, methylation-sensitive restriction enzymes, amplification (e.g., PCR) reagents, probes and/or primers. Quantitative amplification methods (e.g., quantitative PCR or quantitative linear amplification) can be used to quantify the amount of intact DNA within a locus flanked by amplification primers following restriction digestion. Amplifications may be monitored in "real time."

Additional methods for detecting methylation levels can involve genomic sequencing before and after treatment of the DNA with bisulfite. When sodium bisulfite is contacted to DNA, unmethylated cytosine is converted to uracil, while methylated cytosine is not modified. Such additional embodiments include the use of array-based assays and multiplex PCR assays. The multiplex PCR assay may be Patch PCR. PatchPCR can be used to determine the methylation level of a certain CpG loci. Bisulfite Patch PCR enables multiplexed sequencing of promoter methylation across cancer samples.

Alternatively, restriction enzyme digestion of PCR products amplified from bisulfite-converted DNA is used to detect DNA methylation levels. A "MethyLight" assay may be used alone or in combination with other methods to detect methylation level. Briefly, in the MethyLight process, genomic DNA is converted in a sodium bisulfite reaction (the bisulfite process converts unmethylated cytosine residues to uracil). Amplification of a DNA sequence of interest is then performed using PCR primers that hybridize to CpG dinucleotides. By using primers that hybridize only to sequences resulting from bisulfite conversion of unmethylated DNA, (or alternatively to methylated sequences that are not converted) amplification can indicate methylation status of sequences where the primers hybridize. Similarly, the amplification product can be detected with a probe that specifically binds to a sequence resulting from bisulfite treatment of an unmethylated (or methylated) DNA. If desired, both primers and probes can be used to detect methylation status. Thus, kits for use with MethyLight can include sodium bisulfite as well as primers or detectably-labeled probes (including but not limited to Taqman or molecular beacon probes) that distinguish between methylated and unmethylated DNA that have been treated with bisulfite. Other kit components can include, e.g., reagents necessary for amplification of DNA including but not limited to, PCR buffers, deoxynucleotides; and a thermostable polymerase.

In other particular embodiment of the present invention, the primers used comprises the nucleotide sequence SEQ ID NO: 1, SEQ ID NO: 2, SEQ ID NO: 4, SEQ ID NO: 5, SEQ ID NO: 7 or SEQ ID NO: 8.

In other particular embodiment of the present invention, the methylation level of the cytosine residue is measured or determined by one or more methods selected from the list consisting of: methylation-specific polymerase chain reaction, real time methylation-specific polymerase chain reaction, PCR using a methylated DNA-specific binding protein, quantitative PCR, bisulfite pyrosequencing and microarray-based methods.

In other more particular embodiment of the method of the invention, the methylation level is determined by bisulfite pyrosequencing or microarray-based methods.

The inventors have obtained a Random Forest classification model based on prognostic features for the discrimination of benign and malignant thyroid lesions with a training dataset composed of benign and malignant samples (step (a)), using the methylation levels of the three CpG sites, simultaneously.

As the skilled person in the art knows, "random forests model" or "random decision forests" are an ensemble learning method or a machine learning algorithm for classification, regression and other tasks that operates by constructing a multitude of decision trees at training time. For classification tasks, the output of the random forest is the class selected by most trees. For regression tasks, the mean or average prediction of the individual trees is returned. Random decision forests correct for decision trees' habit of overfitting to their training set. In machine learning, a common task is the study and construction of algorithms that can learn from and make predictions on data. Such algorithms function by making data-driven predictions or decisions, through building a mathematical model from input data. These input data used to build the model are usually divided in multiple data sets. In particular, three data sets are commonly used in different stages of the creation of the model: training, validation and test sets.

Particularly, the term "training dataset" used herein refers to a set of examples used to fit the parameters (methylation levels or values) of the model. The current model is run with the training data set and produces a result, which is then compared with the target, for each input vector in the training data set. Based on the result of the comparison and the specific learning algorithm being used, the parameters of the model are adjusted. The model fitting can include both variable selection and parameter estimation.

Once the random forest model is generated by combining the methylation levels or values of the cytosine residues of the 3 CpG sites from the training dataset, a prediction score is determined by introducing the methylation level of the cytosine residues of the 3 CpG sites determined in the sample of interest (the biological sample isolated) (preferably, the methylation levels obtained in step (i)). The term "prediction score" or "malignancy score" used herein refers to the percentage, rate or proportion of benign and malign votes predicted by the random forest model in each of the tree, which can be a number between 0, more benign state, and 1 (or 100%), more malignant state. A prediction or malignancy score of the biological sample isolated is also determined. The prediction or malignancy score is calculated by the following formula:

$$\text{Malignancy score} = 1 - [\text{BN votes} / (\text{BN votes} + \text{M votes})]$$

wherein "BN votes" corresponds with benign votes and "M votes" correspond with malign votes

Once the methylation levels of at least one CpG site or the prediction score have been determined in a sample of the subject, the method of the invention comprises comparing the methylation level of the CpG site obtained in (i) or the prediction score obtained in (c) to a threshold value or a reference value, wherein a methylation level or methylation percentage below or a prediction score above the threshold value is indicative of presence of thyroid carcinoma in the human and the prognosis is negative.

The term "reference value" or "threshold value", used interchangeably in the present invention, refers to a predetermined criteria used as a reference for evaluating the values or data obtained from the samples collected from a subject. The reference value or reference level can be an absolute value; a relative value; a value that has an upper or a lower limit; a range of values; an average value; a median value, a mean value, or a value as compared to a particular control or baseline value. A reference value can be based on an individual sample value, such as for example, a value obtained from a sample from the subject being tested, but at an earlier point in time. The reference value can be based on a large number of samples, such as from population of subjects of the chronological age matched group, or based on a pool of samples including or excluding the sample to be tested. According to the method of the invention, the reference value refers to the average or median value of the methylation levels or methylation percentage of each CpG site of the invention obtained in a set of subjects having non-tumour or non-thyroid carcinoma. Likewise, for the diagnosis of thyroid carcinoma by the random forest model, the reference value can be the prediction or malignancy score which the random forest model is able to discriminate between the benign (non-tumour) and the malignant thyroid carcinoma. Various considerations should be taken into account when determining the reference value. Among such considerations are the age, weight, sex, general physical condition of the patient and the like. For example, equal amounts of a group of at least 2, at least 10, at least 100 to preferably more than 1000 subjects, preferably classified according to the foregoing considerations, for example according to various age categories, are taken as the reference group.

Once the reference value is established, the methylation levels or methylation percentage of each CpG sites of the invention obtained in step (i) or the prediction or malignancy score of the biological sample isolated in step (d) of the method of the invention, can be compared with this reference value, respectively, and thus be assigned a level of "lower" (decrease or below) or "higher" (increase or above) methylation levels or methylation percentage of the CpG sites of the invention or malignancy scores.

Particularly, in the random forest model, the prediction or malignancy score of the biological sample isolated is compared with a threshold value, wherein a prediction score above the threshold value is indicative of presence of thyroid carcinoma (malignant thyroid carcinoma) in the human. Preferably, for determining the prognostic value of the patient of interest is used generic prediction algorithms implemented in the R programming language.

According to the present invention, a methylation level or methylation percentage below the reference o threshold value is indicative of presence of thyroid carcinoma in the human and the prognosis is negative. The methylation levels or methylation percentages are considered to be lower than or below its reference value when it is at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%: at least 85%, at least 90%, at least 95%, at least 100%, at least 110%, at least 120%, at least 130%, at least 140%, at least 150% or more lower than or below the reference value.

In other particular embodiment of the method of the invention, a methylation level or methylation percentage of the CpG site at position 97,858,492-97,858,493 of the human chromosome 13 (cg17154646) and/or the CpG site at position 5,672,830-5,672,831 of the human chromosome 20 (cg10189462) below 50% is indicative of presence of thyroid carcinoma in the human and the prognosis is negative, preferably below 40%, 45% or 30%, more preferably below 25%.

In other particular embodiment of the method of the invention, a methylation level or methylation percentage of the CpG site at position 74,957,673-74,957,674 of the human chromosome 14 (cg21915100) below 70% is indicative of presence of thyroid carcinoma in the human and the prognosis is negative, preferably below 60%. According to random forest model of the present invention, a prediction or malignancy score above the reference o threshold value is indicative of presence of thyroid carcinoma in the human and the prognosis is negative.

In other particular embodiment of the method of the invention, a prediction or malignancy score above 0.25 is indicative of presence of thyroid carcinoma, preferably above 0.3, 0.4, 0.45, 0.5, 0.55, 0.6, 0.65, 0.7, 0.75, 0.8, 0.85, 0.9 or 0.95.

In other particular embodiment of the method of the invention, a prediction or malignancy score below 0.1 is indicative of presence of benign tumor or non-tumour, preferably below 0.15, 0.20 or 0.25.

In order to put the method of the invention into practice, it is necessary to determine the methylation levels of at least one the CpG sites of the invention in a biological sample isolated from a human. As used herein, the term "biological sample" refers to any sample which can be obtained and isolated from the subject and comprises DNA or any sample of biological material derived from a human such as, but not limited to, cellular material, biofluids (eg. blood), faeces, tissue biopsy specimens, surgical specimens or fluid which has been introduced into the body of human and subsequently removed. The term "isolated" means a substance in a form or in an environment that does not occur in nature. In the present invention, the sample is preferably an isolated biological sample, and includes, without limitation to, tissue, whole blood, serum, plasma, body fluid, urine, cells, cell lysates, a supernatant of cell culture or any combination thereof. Preferably, the biological sample is tissue from a biopsy, more preferably from a thyroid biopsy, more preferably fine needle aspiration thyroid biopsy.

The biological sample which is tested according to the method of the present invention may be tested directly or may require some form of treatment prior to testing. For example, a biopsy or surgical sample may require homogenization prior to testing or it may require sectioning for in situ testing of the qualitative expression levels of individual genes. To the extent that the DNA region of interest is present in a biological sample, the biological sample may be directly tested or else all or some of the nucleic acid present in the biological sample may be isolated prior to testing. In yet another example, the sample may be partially purified or otherwise enriched prior to analysis.

Said sample can be obtained by conventional methods, for example biopsy, using methods well known by the persons skilled in related medical techniques. The methods for obtaining a biopsy sample include splitting a tumor into large pieces, or microdissection, or other cell separating methods known in the art. The tumor cells can additionally be obtained by means of cytology through aspiration with a small gauge needle. To simplify sample preservation and handling, samples can be fixed in formalin and soaked in paraffin or first frozen and then soaked in a tissue freezing medium such as OCT compound by means of immersion in a highly cryogenic medium which allows rapid freezing.

In other particular embodiment of the present invention, the biological sample is tissue from a biopsy, preferably from a thyroid biopsy, more preferably from a thyroid nodule biopsy.

By means of the method of the invention, it is able to discriminate between the benign (non-tumour) and malignant (follicular thyroid adenoma or FTA, thyroid carcinoma or FTC and follicular variant of papillary thyroid carcinoma or FVPTC) samples.

Thus, other particular embodiment of the method of the invention relates to *in vitro* diagnosis and/or prognosis of thyroid carcinoma wherein the thyroid carcinoma is follicular thyroid carcinoma (FTC), follicular thyroid adenoma (FTA) or follicular variant of papillary thyroid carcinoma (FVPTC).

Alternative to the above-disclosed method, the present invention also relates to the use of the methylation levels of at least one of the CpG sites of the invention for the *in vitro* diagnosis and/or prognosis of thyroid carcinoma.

Therefore, in another aspect, the present invention relates to the use of the methylation levels of a cytosine residue of at least one CpG site, wherein the CpG site located at position 97,858,492-97,858,493 of the human chromosome 13, position 74,957,673-74,957,674 of the human chromosome 14 and/or position 5,672,830-5,672,831 of the human chromosome 20, preferably the CpG site located at position 97,858,492-97,858,493 of the human chromosome 13, for the diagnosis and/or prognosis of thyroid carcinoma, hereinafter "use of the invention".

In a particular embodiment, the use of the invention comprises the methylation levels of a cytosine residue of the CpG sites, wherein the CpG sites located at position 97,858,492-97,858,493 of the human chromosome 13, position 74,957,673-74,957,674 of the human chromosome 14 and/or position 5,672,830-5,672,831 of the human chromosome 20.

In other particular embodiment of the use of the invention, the cytosine residue of the CpG sites residue is at position 97,858,492 of the positive strand (+) of the human chromosome 13, position 74,957,674 of the negative strand (-) of the human chromosome 14 and/or position 5,672,830 of the positive strand (+) of the human chromosome 20.

All the definitions and particular embodiments disclosed for the method of the invention are applicable to the use of the invention.

The present invention also provides kits for determining the methylation levels of a cytosine residue of at least one of the CpG sites of the invention, so that it is possible to *in vitro* diagnose and/or prognosticate the presence of thyroid carcinoma, as explained in the beginning of the present disclosure.

Thus, in another aspect, the present invention relates to a kit for *in vitro* determination, in a biological sample isolated from a subject, of the methylation levels of a cytosine residue of at least one CpG site wherein the CpG site located at position 97,858,492-97,858,493 of the human chromosome 13, position 74,957,673-74,957,674 of the human chromosome 14 and/or position 5,672,830-5,672,831 of the human chromosome 20, hereinafter "kit of the invention", comprising at least a pair of primers capable of amplifying a fragment containing the CpG site.

In another particular embodiment of the kit of the invention, the pair of primers comprises the nucleotide sequences SEQ ID NO: 1 and SEQ ID NO: 2, SEQ ID NO: 4 and SEQ ID NO: 5 and/or SEQ ID NO: 7 and SEQ ID NO: 8.

In another particular embodiment, the kit of the invention further comprises at least one sequencing primer.

The term "sequencing primer" used herein, refers to the oligonucleotide that triggers the sequencing reaction in the context of a pyrosequencing experiment.

In another more particular embodiment of the kit of the invention, the sequencing primer comprises the nucleotide sequences SEQ ID NO: 3, SEQ ID NO: 6 and/or SEQ ID NO: 9.

In order to put into practice the method of the invention or the use of the invention, reagents for *in vitro* determining the methylation levels of the CpG sites are needed. These agents can be provided in the kit of the invention. Reagents for detection of methylation include, e.g., conversion reagent (sodium metabisulfite), denaturation reagent (sodium hydroxide), binding reagent (guanidinium chloride or guanidinium thiocyanate), DNA protection reagent (tetrahydrofurfuryl alcohol, 6-hydroxy-2,5,7,8-tetramethylchromane-2-carboxylic acid), desulphonation reagent (propan-2-ol, ethanol, sodium hydroxide), wash reagent (sodium azide), Taq DNA polymerase, deoxynucleotides, and/or a methylation-sensitive or methylation-dependent restriction enzyme, and substrate (5' phosphosulfate (APS)), enzyme (DNA polymerase, ATP sulfurylase, luciferase, apyrase, single stranded DNA binding protein) and nucleotides (including alpha-thio triphosphate dATPaS instead of dATP) mixtures for pyrosequencing reactions. The kit can provide reagents for the amplification as polynucleotides designed to hybridize to sequence that is the product of a CpG site of the invention if the sequence is not methylated (one of those biotinylated in its 5' end). The kits can provide solid supports in the form of an assay apparatus that is adapted to use in the assay. The kits may further comprise detectable labels, optionally linked to a polynucleotide, e.g., a probe, in the kit. Other materials useful in the performance of the method can also be included in the kits, including test tubes, transfer pipettes, and the like. The kits can also include written instructions for the use of one or more of these reagents in any of the assays described herein.

In another aspect, the present invention relates to the use of the kit of the invention for the *in vitro* diagnosis and/or prognosis of thyroid carcinoma.

All the definitions and particular embodiments disclosed for the method of the invention are applicable to the kit of the invention and its use.

## DESCRIPTION OF THE DRAWINGS

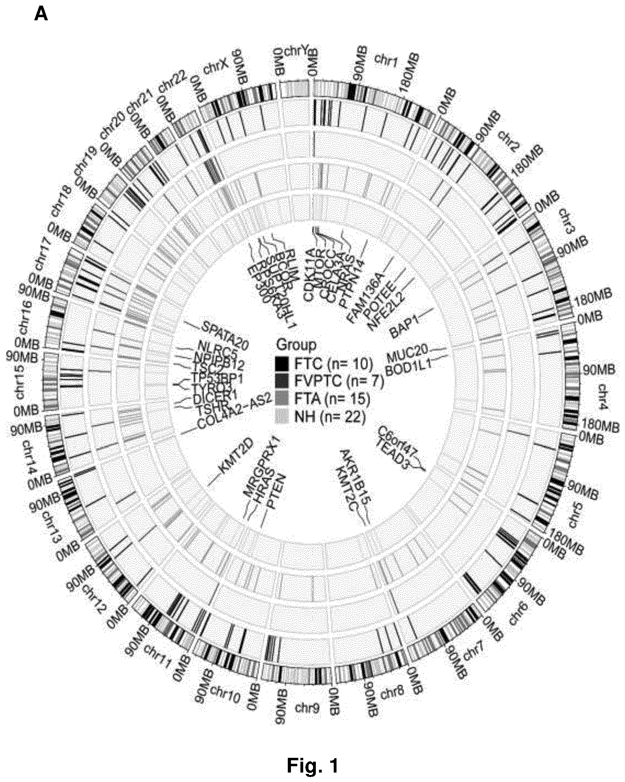

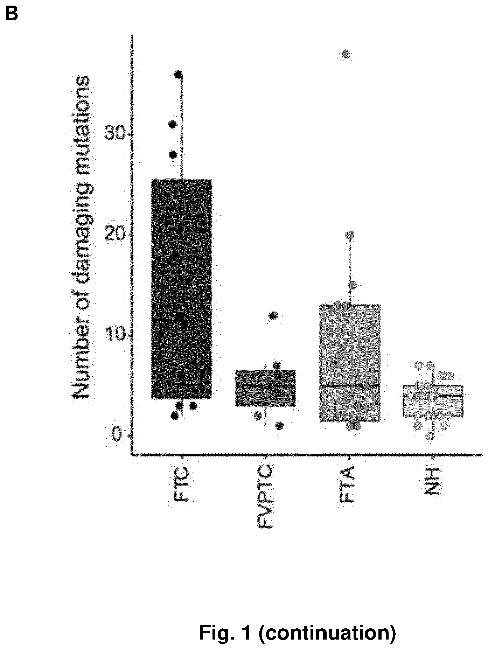

**Figure 1.** Exome sequencing identifies genetic alterations in benign and malignant follicular thyroid lesions. **A)** Circos plot illustrating the mutational landscape of the thyroid samples analysed in this study. Outer track shows the ideogram of human chromosomes (hg38) including the location and staining of known cytobands. Inner tracks show the genomic location of the mutations (lines) identified in the context of FTC, FVPTC, FTA and NH. Genes with recurrent mutations in more than one sample, or genes with known implications for thyroid cancer are highlighted in the inset. The number of cases analysed per cancer category is also shown. **B)** Boxplot indicating the number of damaging mutations observed in the thyroid cancer patients enrolled in this study. Individual dots denote the number of mutations identified in a given sample. For interpretation purposes, samples were grouped according to cancer type.

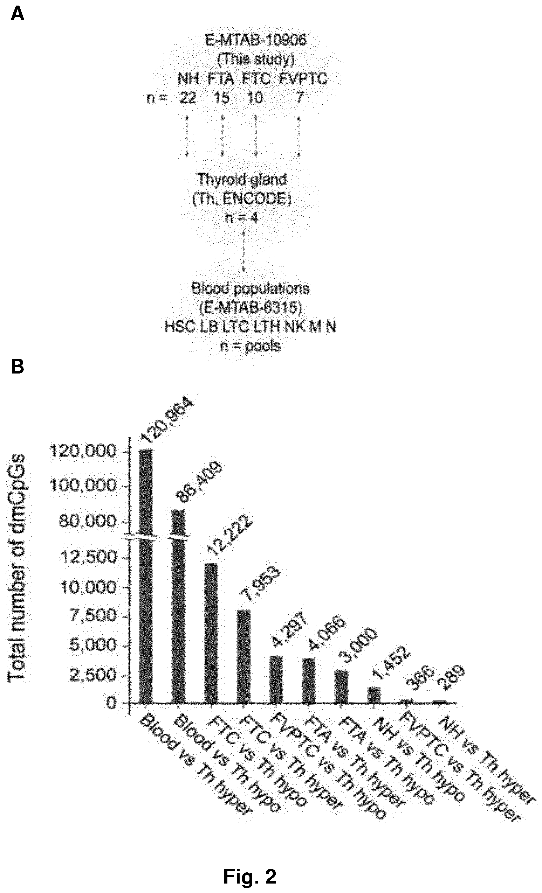

**Figure 2.** Identification of aberrant DNA methylation patterns in follicular thyroid cancer. **A)** Graph illustrating the datasets and the comparisons performed in this study. "n" denotes the number of cases in each of the categories. **B)** Barplots indicating the total number of differentially methylated CpGs (dmCpGs) per category identified in each of the comparisons versus normal thyroid tissue.

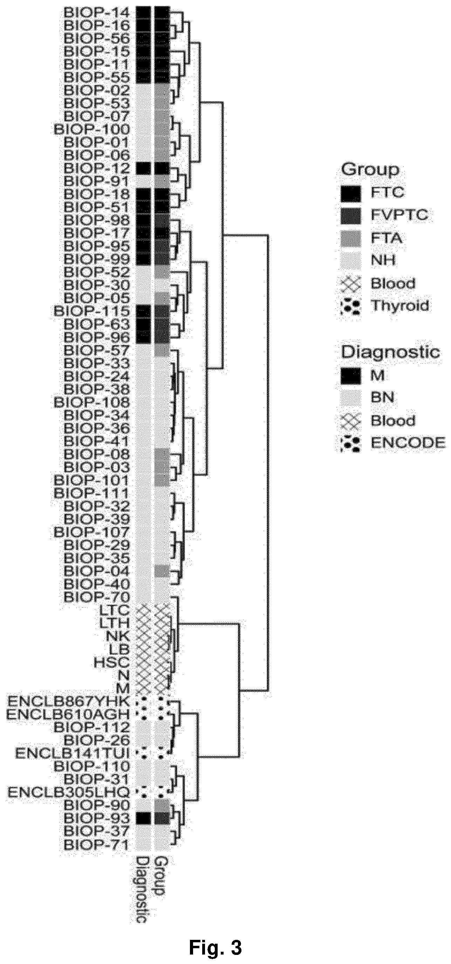

**Figure 3.** Hierarchical clustering analysis of the samples included in this study. Pearson's correlations were calculated using the DNA methylation levels of those 12,845 CpGs which displayed significant differences between NH, FTA, FTC or FVPTC and normal thyroid samples. Blood specific dmCpGs or blood dmCpGs which overlapped in the same direction with any of the aforementioned comparisons were discarded. The legend indicates the cancer category and the diagnosis of each individual sample.

**Figure 4.** Additional information related to the generation of the random forest classifier. **A)** Barplot showing the most informative CpG sites between benign and malignant conditions. Values indicate the number of times that a CpG site was identified as being among the 10 most informative variables using an iterative random forest selection approach (100 different seeds, 5000 trees each). **B)** Line plot illustrating the optimal number of variables (CpGs) required for the development of an accurate model by means of a recursive feature elimination approach. Accuracy reaches its maximum level when 3 features are retained in the model. **C)** Histogram representing the relative importance (Mean decrease Gini) of the three CpG sites included in the final model.

**Figure 5.** A three-CpG epigenetic classifier discriminates between benign and malignant thyroid cancer. **A)** Boxplots indicating the DNA methylation value of three CpG sites across different sets of samples and thyroid cancer categories. This minimal set of CpGs was selected using a random forest approach trained to discriminate between benign (NH) and malignant (FTC, FVPTC) thyroid lesions. The panels show the DNA methylation values of the indicated CpG sites in the context of the EPIC array training dataset. For all the graphs, dots represent individual samples, boxplots were grouped according to cancer type and the number of cases in each category is indicated. Statistical significance between conditions was calculated using a pairwise Wilcoxon rank sum test (* = $p < 0.05$, ** = $p < 0.01$, *** = $p < 0.001$). **B)** Radar plots illustrating the metrics and performance of the proposed three-CpG model in the context of the training dataset.

**Figure 6.** Validation of the three-CpG epigenetic classifier using an independent MethylationEPIC array dataset (GSE121377). **A)** Boxplots indicating the DNA methylation value of the three CpG sites (cg17154646, cg21915100 and cg10189462) in a previous MethylationEPIC study (GSE121377) across different sets of samples and thyroid cancer categories. Statistical significance between conditions was assessed using a pairwise Wilcoxon rank sum test (* = $p < 0.05$, ** = $p < 0.01$, *** = $p < 0.001$). **B)** Radar plots illustrating the metrics and performance of the three-CpG model obtained from GSE121377 in the context of the validation dataset GSE121377. **C)** Boxplots illustrating the DNA methylation values of the indicated CpGs in the context of benign and NIFTP samples from an independent MethylationEPIC array (GSE121377) validation dataset. NIFTP displays features of malignant tumours using the three-CpG random forest classifier. Statistical significance between conditions was calculated using a pairwise Wilcoxon rank sum test (** = $p < 0.01$).

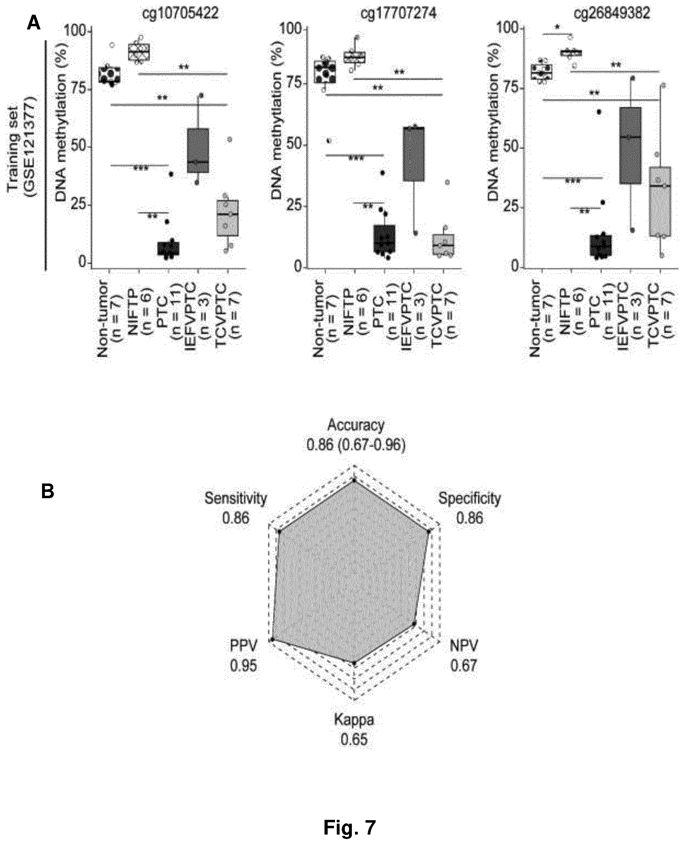

**Figure 7.** Classification of benign and malignant thyroid cancer using previously published epigenetic classification approaches. **A)** Boxplots indicating the DNA methylation value of three CpG sites (cg10705422, cg17707274 and cg26849382) identified in a previous MethylationEPIC study (GSE121377, used as training dataset) across different sets of samples and thyroid cancer categories. A random forest classifier using those three CpG sites was calculated and applied to the GSE121377 training cohort. Statistical significance between conditions was assessed using a pairwise Wilcoxon rank sum test (* = $p < 0.05$, ** = $p < 0.01$, *** = $p < 0.001$). **B)** Radar plots illustrating the metrics and performance of the three-CpG model obtained from GSE121377 in the context of the training dataset GSE121377.

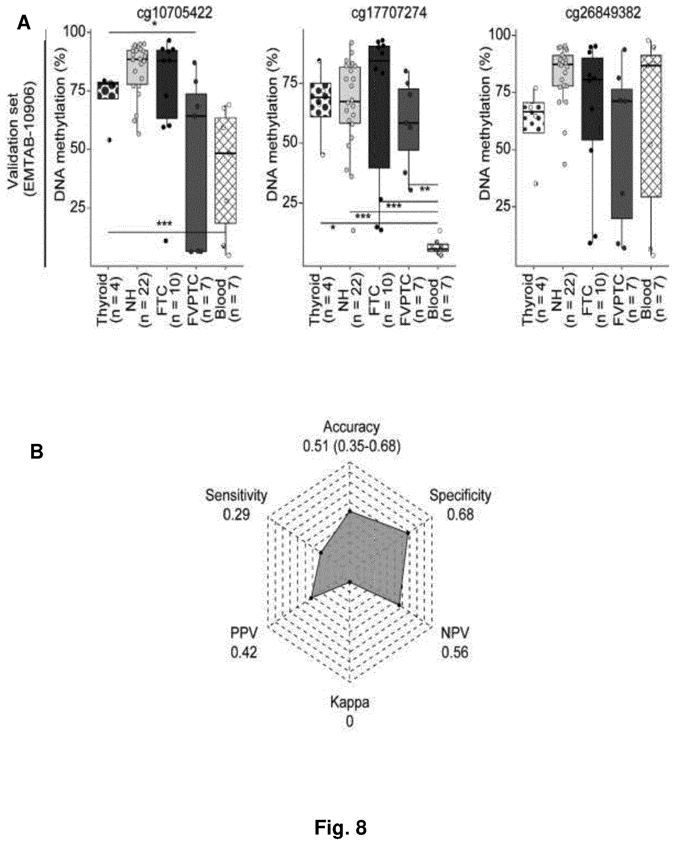

**Figure 8.** Validation of the previously published CpG epigenetic classifier using an independent MethylationEPIC array dataset (E-MTAB-10906). **A)** Boxplots indicating the DNA methylation value of three CpG sites identified in a previous MethylationEPIC study (GSE121377), using E-MTAB-10906 as validation dataset. A random forest classifier using those three CpG sites was calculated and applied to the E-MTAB-10906 validating cohort. Statistical significance between conditions was assessed using a pairwise Wilcoxon rank sum test (* = $p < 0.05$, ** = $p < 0.01$, *** = $p < 0.001$). **B)** Radar plots illustrating the metrics and performance of the three-CpG model obtained from GSE121377 in the context of the training dataset E-MTAB-10906.

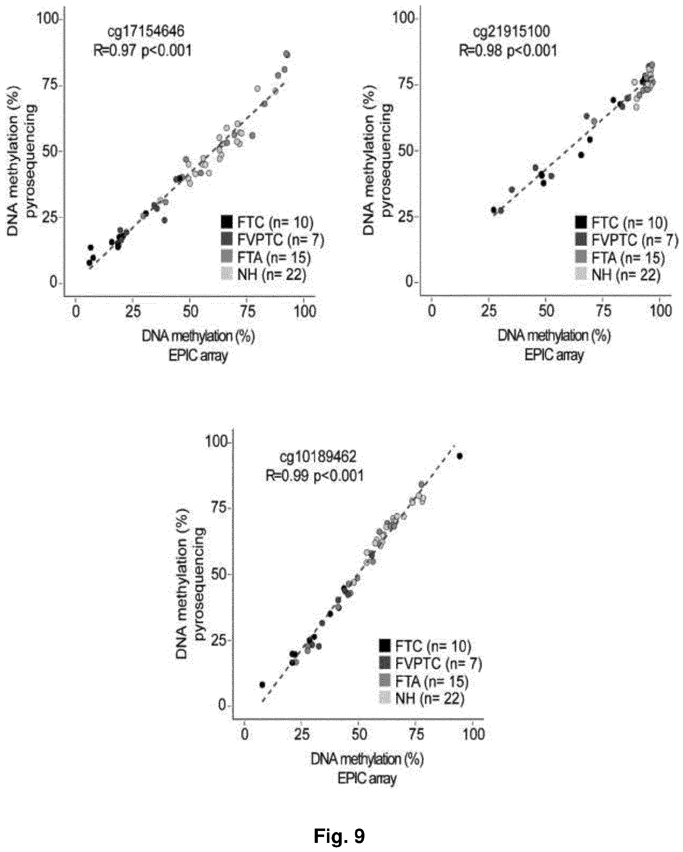

**Figure 9.** Correlation between the Human Methylation EPIC arrays and the DNA pyrosequencing technique. Scatter plots indicating the correlation between DNA methylation Human Methylation EPIC arrays and DNA pyrosequencing of the same set of samples using cg17154646, cg21915100 and cg10189462. Pearson's correlation score, statistical significance and the number of cases included in the different categories are shown.

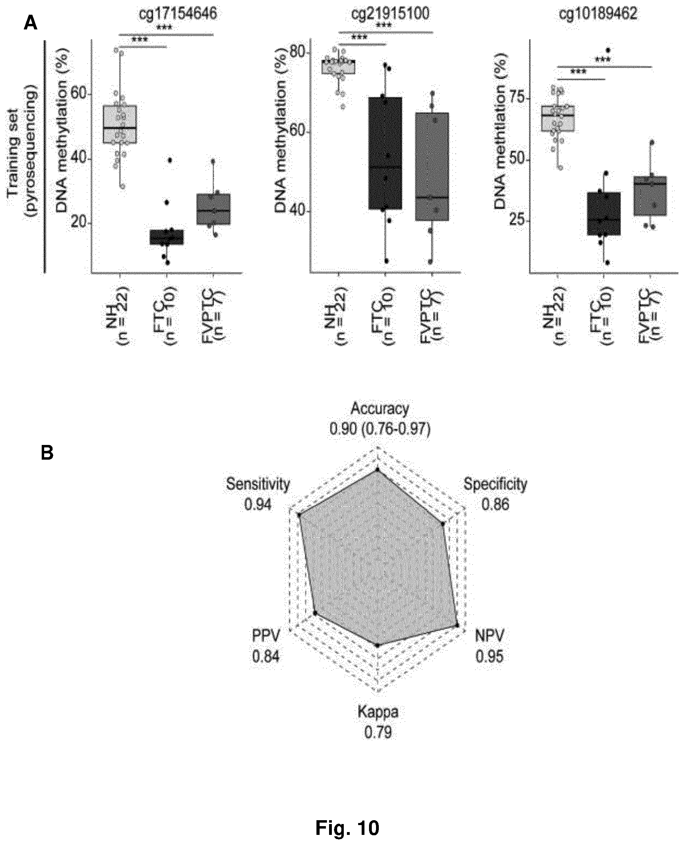

**Figure 10.** Classification of benign and malignant thyroid cancer using the Bisulfite Pyrosequencing technique. **A)** Boxplots indicating the DNA methylation value of three CpG sites across different sets of samples and thyroid cancer categories. This minimal set of CpGs was selected using a random forest approach trained to discriminate between benign (NH) and malignant (FTC, FVPTC) thyroid lesions. Graphs display the DNA bisulphite pyrosequencing methylation values of the indicated CpG sites in the context of the EPIC array training dataset. For all the graphs, dots represent individual samples, boxplots were grouped according to their cancer type and the number of cases included in each category is indicated. Statistical significance between conditions was calculated using a pairwise Wilcoxon rank sum test (* = $p < 0.05$, ** = $p < 0.01$, *** = $p < 0.001$). **B)** Radar plots illustrating the metrics and performance of the proposed three-CpG model in the context of the training dataset.

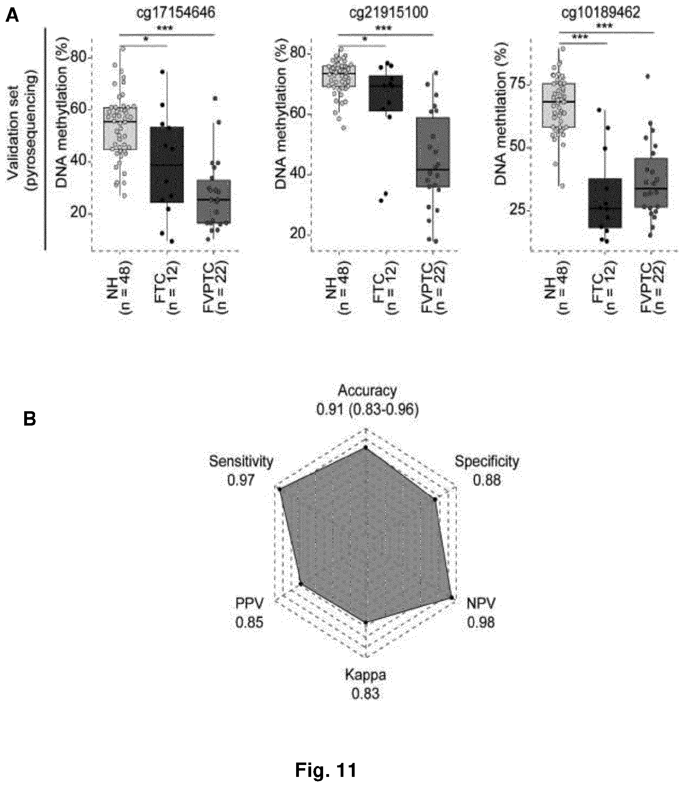

**Figure 11.** Validation of the three-CpG epigenetic classifier using the Bisulfite Pyrosequencing technique. **A)** Boxplots indicating the DNA methylation value of three CpG sites across different sets of samples and thyroid cancer categories. Graphs display the DNA bisulphite pyrosequencing methylation values of the indicated CpG sites in the context of the EPIC array validation dataset. For all the graphs, dots represent individual samples, boxplots were grouped according to their cancer type and the number of cases included in each category is indicated. Statistical significance between conditions was calculated using a pairwise Wilcoxon rank sum test (* = $p < 0.05$, ** = $p < 0.01$, *** = $p < 0.001$). **B)** Radar plots illustrating the metrics and performance of the proposed three-CpG model in the context of the validation dataset.

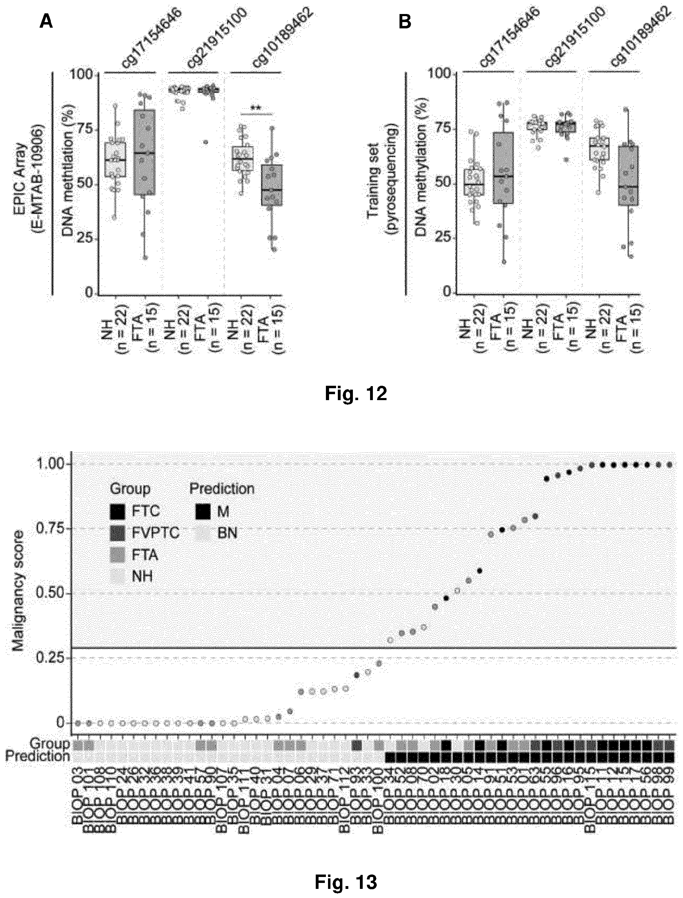

**Figure 12.** Interrogation of the DNA methylation levels of FTA samples in the Training data set using Human Methylation EPIC arrays and Bisulfite pyrosequencing approaches. **A)** Boxplot indicating the DNA methylation values of NH and FTA samples in the context of the three CpG sites used for the modelling of thyroid cancer malignancy using the Human Methylation EPIC platform (E-MTAB-10906). **B)** Boxplot indicating the DNA methylation values of NH and FTA samples in the context of the three CpG sites used for the modelling of thyroid cancer malignancy using the DNA pyrosequencing approach. Statistical significance between conditions was calculated using a pairwise Wilcoxon rank sum test (** = $p < 0.01$).

**Figure 13.** Follicular thyroid adenoma passes through a continuum of cancer malignancy states. Dot plot displaying the inferred cancer malignancy scores of the samples used in the training dataset (bisulfite pyrosequencing results). The cancer type and the diagnostic prediction (benign, BN or malignant, M) of each sample using the three-CpG random forest classifier are indicated.

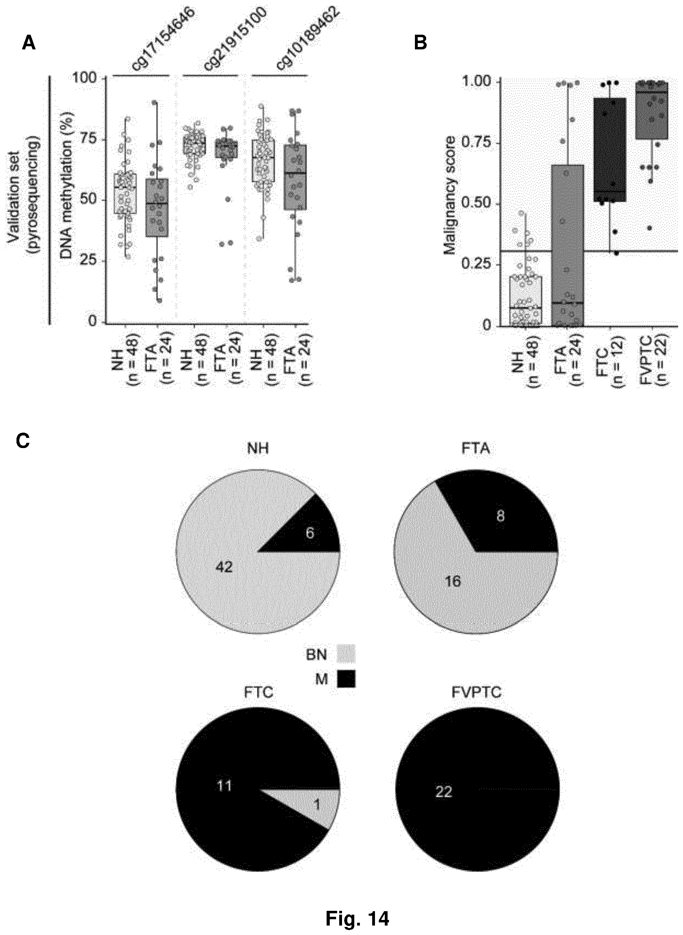

**Figure 14.** Malignancy scores estimated from a validation cohort of Follicular thyroid adenomas and carcinomas. **A)** Boxplot of the DNA methylation values of NH and FTA samples in the context of the three CpG sites in the Validation dataset (pyrosequencing). **B)** Boxplot indicating the inferred cancer malignancy scores of the samples used in the Validation dataset. **C)** Pie-charts showing the number of samples classified as malignant or benign in the different subgroups of thyroid samples of the Validation dataset.

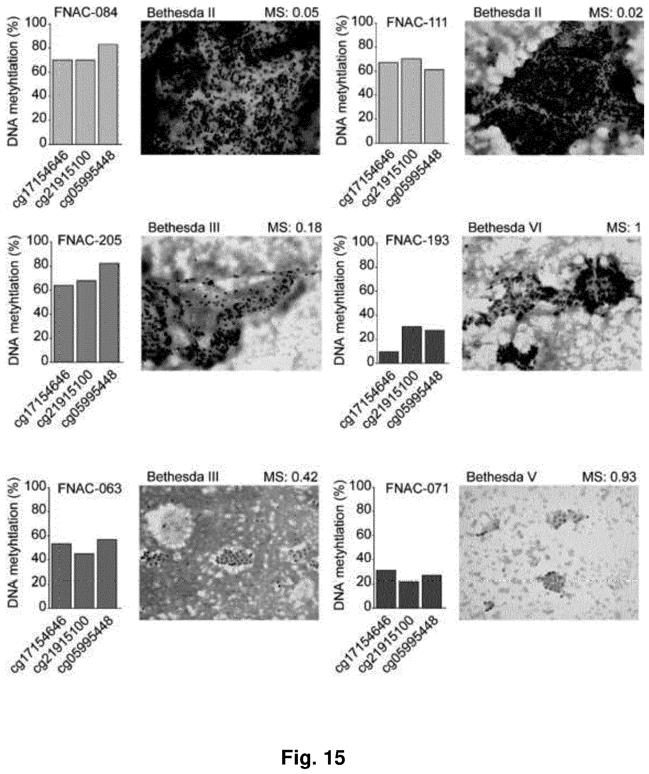

**Figure 15.** Epigenetic classifier discriminates between benign and malignant FNAC samples. Bar plots showing the percentage of methylation of the three CpGs analysed by pyrosequencing in a group of FNAC samples with Bethesda scores between II and VI. The malignancy score assigned by the random forest classifier is indicated above each image. FNAC-084: Haematic background with showing rounded follicular cells without atypia with adequate representation of rounded follicular cells that have macro- and microfollicular distribution. Bethesda II: nodular hyperplasia. FNAC-111: Haematic background with showing rounded follicular cells without atypia with adequate representation of rounded follicular cells that have macro- and microfollicular distribution. Bethesda II: nodular hyperplasia. FNAC-205: Colloid haematic background accompanied by charged macrophages and clusters of predominantly microfollicular cells. No signs of atypia are observed. Given that isolated macrofollicles were observed, we decided in favour of Bethesda III (follicular lesion of uncertain significance). FNAC-193: Haematic background with abundant cellularity, consisting of an epithelial cellularity of follicular appearance with papillary morphology, in which fibrovascular axes can be observed. The follicular cells show atypia due to loss of the nucleus-cytoplasm ratio, hyperchromasia, nuclear irregularity, along with frequent nuclear clefts as well as occasional pseudoinclusions. Bethesda VI: papillary carcinoma.Bar plots showing the methylation values from two FNAC samples obtained from the same patient at two different time points. Left slide: The sample analysed displayed a haematic background, on which we identified slight hypercellularity consisting of follicular cells organized preferentially in macrofollicles, although some microfollicles were also observed. There is atypia due to occasional karyomegaly and moulding. The cytopathological findings are compatible with Bethesda III. Right slide: Haematic background with increased cellularity at the expense of follicular cells with atypia due to karyomegaly, nuclear irregularity, moulding and occasional longitudinal clefts. Bethesda V: cellular atypia suspected to be malignant.

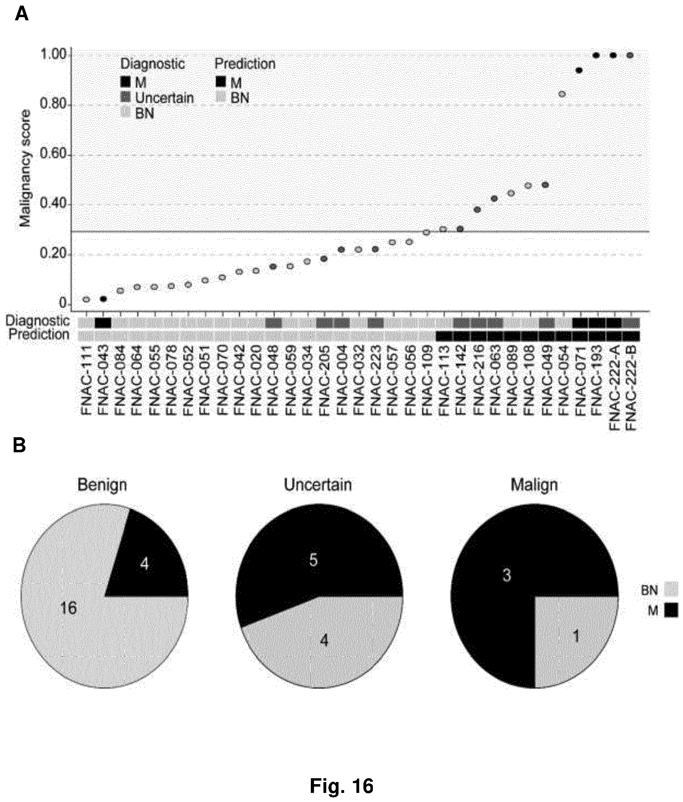

**Figure 16.** Malignancy scores assigned to a cohort of FNAC samples using the three-CpG classifier. **A)** Dot plot showing the inferred cancer malignancy scores of the samples used in the FNACs dataset (using bisulfite pyrosequencing approaches). The cancer type and the diagnostic prediction (benign BN or malignant M) of each sample using the three-CpG random forest classifier are indicated. **B)** Pie charts showing the number of samples classified as malignant or benign in the different subgroups of FNAC samples.

## EXAMPLES

## 1. MATERIAL AND METHODS

### 1.1. Patient Cohorts

Samples and data from patients in this study were retrospectively obtained from the Spanish National Biobanks Network, and samples were processed following standard operating procedures with the appropriate approval of the Ethics and Scientific Committees: CNIO-Biobank, HUB-ICO-IDIBELL (PT17/0015/0024), Principado de Asturias-HUCA Biobank, Hospital de La Princesa Biobank, Hospital Universitario Ramon y Cajal-IRYCIS Biobank (PT17/0015/0010), IDIBGI Biobank (Biobanc IDIBGI, B.0000872), idiPazBiobank (PT17/0015/0025), RBLleida Biobank (B.0000682/PT20/00021), BasqueBiobank, HCSC BioBank B.0000725 (PT17/0015/0040) and BEOCyL Biobank (PT20/00085)-The Institute for Biomedical Research of Salamanca (IBSAL). All the slides corresponding to neighbouring tissue sections were reviewed to verify diagnosis and tumour cell content.

Simultaneous genomic and epigenomic profiling was performed in a cohort (Training set) totalling 54 snap frozen thyroid samples comprising samples of: follicular thyroid adenoma (FTA, n=15), nodular hyperplasia (NH, n=22), follicular thyroid carcinoma (FTC, n=10) and the follicular variant of papillary thyroid carcinoma (FVPTC, n=7). Additionally, a cohort of 106 snap frozen thyroid samples (Validation set) [FTA (n=24), NH (n=48), FTC (n=12) and FVPTC (n=22)] was used to validate the selected DNA methylation markers through pyrosequencing.

To validate the classifier utility in FNAC related material, a third sample cohort was used. A group of FNAC samples (n=33) were prospectively collected by the Pathology Department at the Hospital Universitario Central de Asturias (HUCA, Asturias, Spain). The cytology of each nodule was evaluated and classified according to the Bethesda System for Reporting Thyroid Cytopathology (BSRTC) (2) and the following classifications obtained: Bethesda II, benign (n=20), Bethesda III, atypia of undetermined significance / Follicular lesion of undetermined significance (n=8), Bethesda IV, follicular neoplasia / suspected follicular neoplasia (n=1), Bethesda V, Suspected malignancy (n =2) and Bethesda VI, malignant (n=2). This classification is a diffuse classification and it depends a lot on the pathologist who evaluates it to classify it in one category or another.

### 1.2. DNA isolation

Genomic DNA was purified from fresh-frozen tissues and FNAC samples were taken using a standard phenol-chloroform extraction protocol, which was followed by quantification by Qubit® dsDNA BR Assay in the Qubit® 2.0 Fluorometer (Life Technologies, Carlsbad, CA, USA). Bisulfite modification of DNA was performed with the EZ DNA Methylation Gold kit (Zymo Research, Orange, CA, USA) according to manufacturer's recommendations.

### 1.3. Genomic and Epigenomic Profiling

Whole exome sequencing was performed at Macrogen, Inc (Seoul) using the SureSelect V6 Target enrichment protocol and the Illumina Novaseq 6000 platform (150bp paired-end read mode). The subsequent steps of the exome-seq analyses were performed according to the GATK best practices recommendations using the GATK framework (v.4.1.8). The resulting sequencing reads were aligned to the human reference genome hg38 using Burrows-Wheeler Aligner Bwa-mem (v.0.7.17). SAM files were converted to BAM files using Samtools (v.1.7) and binary files were sorted with the Picard toolkit (v.2.22.8). Polymerase chain reaction duplicates were marked, and a base-quality recalibration step was performed using data from known indels (1000G gold standard indels) and known SNPs (dbSNP138) obtained from the GATK resource bundle. Coverage metrics, sequencing artefacts, pileup summaries and the calculation of sample contamination were performed for downstream purposes. Single nucleotide variations were identified using Mutect2 (v.4.1.8) by means of the patient BAM files mentioned above, the allele frequencies from the Genome Aggregation Database (gnomAD) and a panel of normals (PON) obtained from the 1000 genomes project.

The resulting VCFs were sorted, and a read orientation model was calculated for later use in the variant filtering step. Mutect2 Calls were filtered using the results from the calculated contamination step, the read orientation model, and the list of SureSelect V6 intervals. Simple filters were applied to the resulting VCF files. Variants that passed the standard MuTect2 filters was looked and, for downstream purposes, bcftools (v.1.9) was used to select those entries with a minimum depth coverage > 40 and a minimum allele frequency > 0.15. Filtered VCF files from all the samples were merged using vcftools (v.0.1.16) and the resulting variants were annotated using ANNOVAR (v.2019oct24). Pathogenic variants were defined using non-synonymous SNV, stop gains and frameshift substitutions at exonic, splicing and UTR regions with a MAF < 0.01 using annotations from the 1000 genomes project (version 2015 Aug). The functional effects of these damaging variants were assessed with the Polyphen2, MutationTaster and SIFT algorithms. Frameshift insertions and deletions, as well as those variants identified as damaging mutations in the previous analyses, were considered as potential pathogenic mutations for downstream purposes.

### 1.4. DNA methylation profiling

DNA methylation profiling was performed with Illumina's high content Infinium HumanMethylationEPIC 850K beadchip platform following the Illumina Infinium HD methylation protocol. Array hybridization services were provided by Oxford Genomics Centre (The Wellcome Centre for Human Genetics, University of Oxford, UK). Raw data files (IDAT) from the HumanMethylationEPIC Beadchip platform were processed using R/Bioconductor package minfi (v_1.32.0).

Red and green signals from the raw data were corrected using the ssNOOB algorithm using the default parameters. Probes overlapping genetic variants, probes located in sexual chromosomes, cross-reactive and multimapping probes and probes with at least one sample with a detection p-value>0.01 were discarded from downstream analyses. A Beta-mixture quantile Normalisation method (BMIQ) was applied for the correction of probe bias using the R/Bioconductor package wateRmelon (v.1.30.0). The subsequent B-values and M-values were computed and employed throughout the differential methylation pipeline. To account for potential batch effects or confounding variables, a surrogate variable analysis was performed using the sva algorithm (v.3.35.2). For each comparison, coefficients of the detected surrogate variables (SVs) were estimated using the n.sv function in the context of M-values, and n.sv -1 surrogate variables were included in the definition of the model for the detection of differentially methylated CpGs (dmCpGs).

The statistical significance of the DNA methylation probes was calculated with the moderated t-test implemented in limma (v_3.38.3). A linear model was fitted using methylation level (M-values) as response and sample type (cancer/normal) as the main covariate of interest. In addition, the SVs generated in the surrogate variable analysis were included in the definition of the model. The resulting p-values obtained from the different contrasts were corrected for multiple testing using the Benjamini-Hochberg method. A false discovery rate threshold of 0.05, and an absolute difference between mean DNA methylation values of cases and controls of at least 25% were used for the assessment of significant dmCpGs. However, this threshold was set to 10% in the case of blood versus thyroid gland comparisons in order to discard those probes with minimal fluctuations between different tissue types.

For descriptive purposes, dmCpGs were assigned to their corresponding genomic context or genomic location using the annotation included in the R/BioconductorIlluminaHumanMethylationEPIC.anno.ilm10b2.hg19 package (v_0.6.0). Statistical significance was calculated by means of a two-sided Fisher's test versus an appropriate background which included all filtered CpG probes interrogated by the Methylation EPIC platform.

### 1.5. Random Forest-based classification system

The random-forest based classifier was built using the R/CRAN random forest package (v.4.6.14) and involving a training set of 22 benign (22 NH) and 17 malignant (10 FTC, 7 FVPTC) samples, and the input of the filtered dmCpGs from the previous step.

To focus on the most informative set of epigenetic alterations identified in the different thyroid lesion subtypes, initially the set of 12,845 dmCpGs resulting from the sum/intersection of the NH, FTA, FTC and FVPTC versus normal thyroid gland comparisons was considered, excluding those changes shared in terms of significance and direction with blood tissue. For correct model generation, the number of input variables was reduced using an additional filter of highly correlated dmCpGs (cut-off 0.9) with the R/CRAN Caret Package (v.6.0.86). A random-forest based classifier was built using the R/CRAN random Forest package (v.4.6.14) which included a training set of 22 benign (22 NH) and 17 malignant (10 FTC, 7 FVPTC) samples, and the input of the 8,214 filtered dmCpGs from the previous step. By means of a bootstrap, iterative randomization with a seed function implemented on the training set, a total of 100 models was trained using the random Forest function with its default parameters, except for the number of trees, which was set to 5,000 per model, and the cut-off value for the prediction of benign class was set to 0.7 of the votes for better sensitivity/specificity.

For each model, variable importance was ranked according to the model's Mean Decrease Gini, and the top 10 most important variables were selected for downstream filtering purposes. For final variable selection, the cross-validation method (10-fold) was used, along with the recursive feature elimination method within the R/CRAN Caret package and the rfe algorithm, using the 10 variables with the highest number of top informative observations across models.

The best model performance on the training set was achieved using a minimal number of 3 variables (cg17154646, cg21915100 and cg10189462 probes). Malignancy score for a given sample was calculated based on the ratio of malignant votes over the total number of votes (5000) predicted using the model mentioned earlier, the matrix with the DNA methylation values of the three CpG sites and the predict function of the R stats package (v.4.0.2).

**1.6. Bisulfite Conversion and Pyrosequencing assays**

The DNA methylation pattern of selected CpGs identified by microarray analysis was evaluated by bisulfite pyrosequencing. The set of primers for PCR amplification and sequencing were designed by PyroMark Assay Design Software (v. 2.0.01.15). Primer sequences were designed to hybridize with CpG free sites to ensure methylation-independent amplification. Briefly, 2 $\mu$L of the bisulfite-modified DNA was amplified by PCR with the specific primer set (Table 1).

*Table 1. Information related to the genomic coordinates of the CpG sites interrogated in the patient samples and the pyrosequencing primers used for this experiment. FW: direct or forward primer, RW: reverse primer, SEQ: sequencing primer, BIOT: biotin (linked to primer at first position). The optimal hybridization temperature used for the PCR reaction step and the expected length of the PCR amplicons for quality control purposes are also indicated.*

| PRIMER SET | CpG Position | Primer | SEQUENCE (5'-3') | Annealing Temperature | Amplicon Length (bp) |
|---|---|---|---|---|---|
| MBNL2 assay | chr13: 97858492-97858493 | FW | AAGATTGAAAGAATTGG GTATGGAA (SEQ ID NO: 1) | 63 ºC | 120 |
| | | BIOT-RW | TAACCCAACAAAATTTAC TCCCCTATC (SEQ ID NO: 2) | | |
| | | SEQ | CCTCAAAAAACAAACCAC (SEQ ID NO: 3) | | |
| NPC2 assay | chr14: 74957673-74957674 | BIOT-FW | AAGGTGGGTGTAGAGTG GAATAT (SEQ ID NO: 4) | 60ºC | 325 |
| | | RW | ATAACTCATTCCATCCTC ACAAACTATC (SEQ ID NO: 5) | | |
| | | SEQ | GGAAATTTAAAGTGGTTT ATAAAG (SEQ ID NO: 6) | | |
| GPCPD1 assay | chr20: 5672830-5672831 | FW | TGAAGGGAGTGAGGGTT TATTAG (SEQ ID NO: 7) | 60ºC | 114 |
| | | BIOT-RW | CTAACCACTTCACAAAAC TCTTTTACTTAT (SEQ ID NO: 8) | | |
| | | SEQ | AGATTAGAGTAGGTGTT G (SEQ ID NO: 9) | | |

Next, pyrosequencing was performed using Pyromark Q24 reagents (Qiagen) by immobilizing 20 $\mu$L of PCR product with 2 $\mu$L of Streptavidin Sepharose High Performance (GE Health-care Bio-Sciences, Uppsala, Sweden) for 10 min at 80ºC, and then annealing the resulting sample with a sequencing primer. Samples were processed using the PyroMark Q24 system (Qiagen, Valencia, CA) and the results were analysed with the PyroMark analysis software (Qiagen).

**1.7. Data availability**

The raw exome-seq data (Fastq files) corresponding to human thyroid lesions have been deposited in the European Genome-Phenome Archive (EGA) under the accession number EGAS00001005561 (Dataset EGAD00001008022). Raw HumanMethylationEPIC data including IDAT files from human thyroid lesions (NH, FTA, FTC and FVPTC, training set) have been deposited in ArrayExpress under the accession number E-MTAB-10906. Raw HumanMethylationEPIC data (IDATs) from different blood cell populations (haematopoietic stem cells, HCS; B-cells, LB; T-cells (CD8), LTC; T-cells (CD4), LTH; natural killer cells, NK; monocytes, M; neutrophils; N) were obtained from the ArrayExpress entry E-MTAB-6315. Raw HumanMethylationEPIC data (IDATs) from 4 control human thyroid glands were obtained from the ENCODE project (phase 3) with the accession numbers ENCLB141TUI, ENCLB867YHK, ENCLB610AGH and ENCLB305LHQ. For validation purposes, raw HumanMethylationEPIC data (IDATs) was obtained from a recent work focused on the identification of thyroid cancer biomarkers with the GEO dataset entry GSE121377 (Park JL., et al 2020, Comprehensive DNA Methylation Profiling Identifies Novel Diagnostic Biomarkers for Thyroid Cancer. Thyroid. Mary Ann Liebert Inc.; 30:192-203).

**2. RESULTS**

**2.1. The mutational landscape of thyroid lesions with follicular like features**

The accumulation of genetic and epigenetic alterations plays a key role in the development of oncogenic signatures in human cancers. To identify molecular alterations that may explain the tumorigenic potential of benign and malignant thyroid lesions with a follicular phenotype, which are frequently associated with cytological indeterminate thyroid nodules, first the mutational landscape of FTC and FTA were explored using a whole exome-seq (WES) approach. To cover the full spectrum of follicular-like thyroid lesions, biopsies from 10 FTC, 7 FVPTC, 15 FTA and 22 NH were analysed with a median depth of coverage of 132 X. This analysis revealed the presence of 148 FTC, 36 FVPTC, 132 FTA and 76 NH potentially damaging mutations in the Training set (Figure 1A). FTC and NH samples displayed, respectively, the highest and the lowest number of damaging mutations per sample (Figure 1B), and an intermediate, variable number of mutations were identified in FTA patients. Interestingly, FVPTCs did not display as many damaging mutations as their FTC counterparts (median 11.5 versus 5, respectively), suggesting that the molecular mechanisms governing each thyroid carcinoma subtype may result in a different tumour mutational burden. Thus, it was sought to determine whether these genetic alterations could act as a good proxy for classifying the different follicular thyroid lesions according to their potential prognostic status. Recurrent mutations in classical thyroid cancer genes were observed in the Training set, including TSHR, TP53, PTEN, DICER1 and RAS genes. However, given the high genetic variability observed in the thyroid lesions mentioned earlier, it was unable to define significant mutational clusters associated with malignancy status, or any of the diagnostic groups, indicating that the molecular alterations encoded in the genome are complex and suboptimal for the appropriate prognostic estimation of follicular thyroid lesions.

**2.2. Epigenomic Alterations in follicular-like thyroid lesions**

To overcome the prognostic limitations of the genetic analyses, a comprehensive epigenomic analysis of the same follicular-like thyroid lesions was performed using high content DNA methylation arrays. The ENCODE thyroid gland dataset (n=4) as a universal was used, normal reference epigenome to identify epigenomic alterations in the Training set (15 FTA, 10 FTC, 7 FVPTC and 22 NH fresh frozen thyroid samples). In addition, and as a control for the thyroid-specific changes, a differential methylation analysis of thyroid gland versus a pool of different haematopoietic cell populations was performed (Figure 2A). The global DNA methylation status of the different thyroid sample subtypes oscillated between median DNA methylation scores of 0.78 and 0.82, with the pool of blood samples displaying a substantial hypermethylation scenario (median score 0.85). Blood versus thyroid gland epigenomes displayed the greatest number of differentially hyper-and hypomethylated CpGs as compared to the rest of the comparisons. Interestingly, the number of epigenetic alterations was correlated with the prognostic status of the thyroid lesions, with FTC and NH having, respectively, the highest and the lowest number of dmCpGs (Figure 2B).

To avoid any potential confounding effects caused by, for example, immune cell infiltrates, the inventors focused solely on those specific dmCpGs not shared, or at least displaying opposite directionality, between the blood samples and any of the previously mentioned thyroid lesion comparisons, resulting in the identification of 12,845 dmCpG sites.

Hierarchical clustering of the discovery cohort using pairwise correlations and the DNA methylation information encoded in these highly specific sites revealed the presence of different clusters. Clustering analysis was useful in discriminating the malignancy of a thyroid lesion, a clear differentiation between benign (NH, Blood, Thyroid, bottom) and malignant (FTC, FVPTC, top) samples being evident (Figure 3). In addition, it was observed that samples corresponding to the same thyroid lesion subtype tended to cluster together, except for the FTA group, which displayed a disperse pattern across multiple clusters. This issue reinforces the hypothesis that FTA samples are not a defined entity and may represent a continuum towards malignant states, also at the molecular level, being potentially associated with either a benign or malignant prognosis depending on their intrinsic epigenomic features.

## 2.3. Development of a minimal epigenetic classification system of thyroid lesion malignancy

Given that thyroid lesion heterogeneity has been accurately identified using previous epigenetic approaches, epigenetic features may provide ideal terrain for the identification of molecular biomarkers of the current malignancy state of a tumour. Thus, the 12,845 dmCpG sites obtained in the previous restrictive filtering approach were used to build a robust Random Forest classification model based on prognostic features for the correct discrimination of benign and malignant thyroid lesions. The training dataset was composed of 22 benign (NH, n=22) and 17 malignant (FTC, n=10; FVPTC, n=7) samples. The FTA samples (n=15) were discarded from the initial analysis because their dispersed nature could have led to confounding effects.

By selecting the most informative and robust dmCpGs (Figure 4 A, B and C), a minimal classification system was obtained constituted by three CpG probes (cg17154646, cg21915100 and cg10189462) located, respectively, in the vicinities of the MBNL2, NPC2 and GPCPD1 genes.

The significant discrimination of benign from malignant samples in the training dataset was observed at the DNA methylation level using any one of the individual CpG sites (Figure 5A). When all three of these CpGs were simultaneously implemented in the Random Forest classifier using a cross-validation approach, an accuracy of 0.95, with a sensitivity of 1.00 (17 of 17 malignant samples), and a specificity of 0.90 (20 out of 22 benign samples) in the training set were obtained (Figure 5B).

This minimal classification system was subsequently validated by means of different orthogonal approaches. First, the inventors collected data from the above-described study *Park JL., et al 2020* focused on the identification of diagnostic biomarkers of thyroid cancer using a similar DNA methylation platform (GSE121377). The three-CpG classification system was able to discriminate between the benign (non-tumour, n=7) and the malignant papillary thyroid carcinoma subtypes identified in the study (Classic Papillary Thyroid Cancer, PTC, n=11; Invasive encapsulated follicular variant of PTC, IEFVPTC, n=3; Tall Cell Variant Papillary Thyroid Cancer, TCVPTC, n=7) samples from the GSE121377 dataset (Figure 6A), with a classification accuracy of 0.96, a sensitivity of 0.95 and a specificity of 1.00 (Figure 6B). Interestingly, Non-Invasive Follicular Thyroid neoplasms with Papillary-like nuclear features samples (NIFTP, n=6) were consistently classified as malignant by our Random Forest classifier (Figure 6C), suggesting that despite being encapsulated, their molecular content closely resembles the epigenomic features of malignant cells.

Additionally, to test the discrimination efficacy of the different classification systems proposed in the literature, an analogous analysis on the DNA methylation array dataset was performed using the epigenetic alterations described by *Park JL., et al 2020.* For the estimation of malignancy status, a Random Forest classifier was trained using the information obtained from the cg10705422, cg17707274 and cg26849382 probes used in the GSE121377 dataset. A statistically significant capacity to discriminate between benign and malignant thyroid lesions was independently accomplished with any one of the three CpG sites used individually in the training dataset (Figure 7A). In the absence of the NIFTP group, the implementation of these CpGs in a Random Forest model yielded a classification accuracy of 0.86, with a sensitivity of 0.86 and a specificity of 0.86 (Figure 7B).

It is worth noting the reduced performance of the three-CpG model obtained in *Park JL., et al 2020* in terms of discriminating the malignancy status of follicular-like lesions from the DNA Human Methylation EPIC dataset as compared to the three-CpG model (Figure 8A and 8B, accuracy 0.51, sensitivity 0.29, specificity 0.68). These results indicate that the three-CpG model outperforms previous epigenetic classification systems of thyroid cancer malignancy and extends the use of this minimal three-CpG classifier to other contexts, including those thyroid lesions with papillary and/or follicular-like features.

Finally, to demonstrate the applicability of the classification system in other cost-effective experimental conditions, the results obtained with cg17154646, cg21915100 and cg10189462 were validated using classical pyrosequencing assays. A strong significant correlation between the DNA methylation levels obtained from either DNA methylation arrays or the DNA pyrosequencing platform (average correlation coefficient=0.98, p<0.001) was observed (Figure 9), indicating that the DNA methylation status of these samples can be also monitored by a less expensive and faster experimental approach.

To validate the application of the three-CpG classifier in this scenario, the Random Forest model was rebuilt using the pyrosequencing DNA methylation values obtained in the training dataset. Cross-validation of this model yielded a similar classification accuracy (0.90) as with the aforementioned Human Methylation EPIC arrays, with sensitivity, and specificity values of 0.94 and 0.86 respectively (Figure 10A and 10B).

To corroborate these observations, one last round of validation of the three-CpG classifier was performed by means of DNA pyrosequencing assays using an independent cohort of 48 NH, 12 FTC and 22 FVPTC samples (Validation set). Using the Random Forest model built upon the three CpG sites (cg17154646, cg21915100 and cg10189462), the malignancy status of the validation dataset was predicted with a classification accuracy of 0.91, a sensitivity of 0.97 (33 of 34 malignant samples) and a specificity of 0.88 (42 out of 48 benign samples) (Figure 11A and 11B), confirming the discrimination power of the minimal epigenetic classification system.

## 2.4. Prognostic discrimination of FTA samples according to epigenetic biomarkers

Initial observations indicated that the epigenetic features displayed by FTA samples represent a continuum between benign and malignant states. At the level of the three key CpG sites, it was not observed significant differences in the methylation values of NH and FTA subtypes in the Training data set, nor in the context of the DNA methylation arrays (Figure 12A) or in pyrosequencing experiments (Figure 12B), the FTA samples in most instances being dispersed across a wide continuous DNA methylation range. Indeed, the inclusion of these FTA cases (n=15) in the Random Forest classifier resulted in a decrease in the performance of the model due to the incorrect classification of some samples as malignant tissue. This result suggests that despite FTAs having classically been identified as benign thyroid lesions, a proportion of FTA samples may have acquired molecular alterations that confer on them a potential malignant phenotype.

Thus, it was hypothesized that the three-CpG classification system may provide some clues in relation to the malignancy status of a given FTA sample based on the proportion of benign/malignant votes calculated during the prediction step. Interestingly, most of the NH samples from the Training dataset, which were originally classified as benign tissue, displayed malignancy scores (MS) below 0.3 (Figure 13) while most malignant FTC and FVPTC samples displayed malignancy predictions above this. The malignancy score of FTA samples using this classification system varied between 0 and 0.76 (Figure 13), in agreement with the dispersion observed previously at the epigenomic and the single CpG level. This result indicates that at least 40% of the FTA samples (6 out of 15) can be accurately classified as truly benign cases (malignancy score below 0.05) and suggests that a significant number of FTA patients do not exhibit malignant features at the molecular level and may benefit from not undergoing unnecessary thyroidectomies.

To verify these observations in an independent cohort, these data were validated using the 24 FTA samples from the Validation dataset using pyrosequencing assays. Again, no significant differences were observed between NH and FTA samples in the three CpGs analysed (Figure 14A). Most NH samples displayed malignancy scores below 0.3 (42 of 48), while most FTC and FVPTC samples had malignancy scores above this cut-off. The malignancy scores of FTA samples ranged between 0 and 1 (Figure 14B, C). As can be observed in Figure 14B, from a total of 72 benign samples (48 NH and 24 FTA), at least 80% of the benign samples (n=58, 42 NH and 16 FTA) were correctly diagnosed with our three-CpG classifier, while one third of the FTA samples exhibited clear malignant features, indicating that machine learning-based classification could assist in the prioritization of surgical interventions, particularly in those cases associated with cytologically indeterminate thyroid nodules.

## 2.5. Validation of the epigenetic classification system of thyroid lesion malignancy in thyroid nodules (FNACs)

Next, the diagnostic capacity of our Random Forest classification system was tested in a panel of non-invasive FNAC samples subjected to DNA pyrosequencing assays. First, the methylation status of the three CpGs in a group of 4 FNACs classified as Bethesda II (n=2), Bethesda III (n=1) and Bethesda VI (n=1) was evaluated. All these FNACs were correctly diagnosed by the model (Figure 15), and the malignancy scores predicted by the algorithm agreed with the Bethesda status assigned by clinicians, which was further confirmed through a second FNAC or by surgery.

In addition, the diagnostic potential of the classifier was demonstrated using two FNAC samples obtained from the same patient at different time points of the disease (Figure 15). The first sample, which was initially classified as Bethesda III, yielded a malignancy score of 0.42. Interestingly, a second FNAC obtained from same thyroid nodule 19 days later displayed a malignancy score of 0.93, and after the surgical procedure the patient was finally diagnosed as having FVPTC. These results highlight the usefulness of the classification system in FNAC samples as it provides malignancy scores that are highly correlated with the grade and aggressiveness of a given tumour. Finally, the abovementioned analyses were extended to a cohort of 29 additional FNAC samples (total n=33). Predictions obtained with the three-CpG model (Figure 16A and 16B) resulted in the correct classification of 75% (3 out of 4) of the malignant samples (Bethesda V and VI) and 80% (16 out of 20) of conclusively benign cytology cases (Bethesda II). In the uncertain FNACs group (Bethesda III and IV), 5 samples were classified as malignant, and those were post-surgically confirmed as: PTC (n=1), FVPTC (n=2), FTA (n=1) and Papillary thyroid micro carcinoma (PTMC, n=1), and the diagnosis of the 4 uncertain FNACs classified as benign was also confirmed after surgery.

## Claims

1.    An *in vitro* method for the diagnosis and/or prognosis of thyroid carcinoma comprising:

(i) determining, in a biological sample isolated from a human, the methylation level of a cytosine residue of at least one CpG site, wherein the CpG site is located at position 97,858,492-97,858,493 of the human chromosome 13, position 74,957,673-74,957,674 of the human chromosome 14 and/or position 5,672,830-5,672,831 of the human chromosome 20, and

(ii) comparing the methylation level of the CpG site obtained in (i) to a threshold value, wherein a methylation level below the threshold value is indicative of presence of thyroid carcinoma in the human;

and/or

applying a machine learning algorithm, comprising the steps of:

(a) determining, in a training dataset, the methylation level of the cytosine residues of 3 CpG sites located at position 97,858,492-97,858,493 of the human chromosome 13, position 74,957,673-74,957,674 of the human chromosome 14 and position 5,672,830-5,672,831 of the human chromosome 20;

(b) generating a random forest model, which combines the methylation levels of the cytosine residues of the 3 CpG sites from the training data set, by generating of a total of 5000 trees per model with a cut-off value for a benign set of 0.7 of the total votes;

(c) determining a prediction score of the biological sample isolated, being the prediction score a percentage of benign and malign votes predicted by the random forest model in each of the tree, by introducing the methylation levels of the cytosine residues of the 3 CpG sites determined of the biological sample, and;

(d) comparing the prediction score obtained in (c) with a threshold value, wherein a prediction score above the threshold value is indicative of presence of thyroid carcinoma in the human.

2. The method according to claim 1 comprises determining the methylation level of a cytosine residue of the CpG sites located at position 97,858,492-97,858,493 of the human chromosome 13, position 74,957,673-74,957,674 of the human chromosome 14 and/or position 5,672,830-5,672,831 of the human chromosome 20, preferably of the CpG sites located at position 97,858,492-97,858,493 of the human chromosome 13.

3. The method according to claim 1 or 2 wherein the cytosine residue of the CpG sites residue is at position 97,858,492 of the positive strand (+) of the human chromosome 13, position 74,957,674 of the negative strand (-) of the human chromosome 14 and/or position 5,672,830 of the positive strand (+) of the human chromosome 20.

4. The method according to any one of claims 1 to 3, wherein the biological sample is tissue from a biopsy, preferably from a thyroid biopsy.

5. The method according to any one of claims 1 to 4, wherein the thyroid carcinoma is follicular thyroid carcinoma (FTC), follicular thyroid adenoma (FTA) or follicular variant of papillary thyroid carcinoma (FVPTC).

6. The method according to any one of claims 1 to 5, wherein the methylation level of step (i) is determined by bisulfite pyrosequencing or microarray-based methods.

7. Use of the methylation levels of a cytosine residue of at least one CpG site, wherein the CpG site is located at position 97,858,492-97,858,493 of the human chromosome 13, position 74,957,673-74,957,674 of the human chromosome 14 and/or position 5,672,830-5,672,831 of the human chromosome 20, for the *in vitro* diagnosis and/or prognosis of thyroid carcinoma.

8. Use according to claim 7 of the methylation levels of a cytosine residue of the CpG sites wherein the CpG sites are located at position 97,858,492-97,858,493 of the human chromosome 13, position 74,957,673-74,957,674 of the human chromosome 14 and/or position 5,672,830-5,672,831 of the human chromosome 20.

9. Use according to claim 7 or 8 wherein the cytosine residue of the CpG sites residue is at position 97,858,492 of the positive strand (+) of the human chromosome 13, position 74,957,674 of the negative strand (-) of the human chromosome 14 and/or position 5,672,830 of the positive strand (+) of the human chromosome 20.

10. A kit for *in vitro* determination, in a biological sample isolated from a subject, of the methylation levels of a cytosine residue of at least one CpG site wherein the CpG site is located at position 97,858,492-97,858,493 of the human chromosome 13, position 74,957,673-74,957,674 of the human chromosome 14 and/or position 5,672,830-5,672,831 of the human chromosome 20 comprising at least a pair of primers capable of amplifying a fragment containing the CpG site.

11. The kit according to claim 10 wherein the pair of primers comprises the nucleotide sequences SEQ ID NO: 1 and SEQ ID NO: 2, SEQ ID NO: 4 and SEQ ID NO: 5 and/or SEQ ID NO: 7 and SEQ ID NO: 8.

12. The kit according to claim 10 or 11 further comprises at least one sequencing primer.

13. The kit according to claim 12 wherein the sequencing primer comprises the nucleotide sequences SEQ ID NO: 3, SEQ ID NO: 6 and/or SEQ ID NO: 9.

14. Use of a kit according to any of claims 10 to 13 for the *in vitro* diagnosis and/or prognosis of thyroid carcinoma.

**A**

**Fig. 1**

B

**Fig. 1 (continuation)**

**A**

**B**

**Fig. 2**

**Fig. 3**

**A**

**B**

**C**

Fig. 4

Fig. 5

Fig. 6

**Fig. 7**

**Fig. 8**

Fig. 9

**Fig. 10**

EP 4 234 720 A1

Fig. 11

**Fig. 12**

**Fig. 13**

**Fig. 14**

**Fig. 15**

**A**

**B**

Benign        Uncertain        Malign

**Fig. 16**

Europäisches
Patentamt

European
Patent Office

Office européen
des brevets

## EUROPEAN SEARCH REPORT

**Application Number**

EP 22 38 2165

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/204457 A1 (CATHOLIC UNIV KOREA IND ACADEMIC COOPERATION FOUNDATION [KR] ET AL.) 8 October 2020 (2020-10-08) * claim 1 * | 1-14 | INV. C12Q1/6886 |
| A | ABDUL A QURESHI ET AL: "Genomic differences in benign and malignant follicular thyroid tumours using 1-Mb array-comparative genomic hybridisation", EUROPEAN ARCHIVES OF OTO-RHINO-LARYNGOLOGY ; AND HEAD & NECK, SPRINGER, BERLIN, DE, vol. 270, no. 1, 13 April 2012 (2012-04-13), pages 325-335, XP035157649, ISSN: 1434-4726, DOI: 10.1007/S00405-012-2017-4 * the whole document * | 1 | |
| A | EP 1 942 197 A1 (EPIGENOMICS AG [DE]) 9 July 2008 (2008-07-09) * SEQ ID NO:27766 from Patent EP1942197 comprises SEQ ID NO:3 of the present application * | 1 | |

**TECHNICAL FIELDS SEARCHED (IPC)**

C12Q

The present search report has been drawn up for all claims

| Place of search | Date of completion of the search | Examiner |
|---|---|---|
| The Hague | 4 August 2022 | Gabriels, Jan |

## ANNEX TO THE EUROPEAN SEARCH REPORT
## ON EUROPEAN PATENT APPLICATION NO.

EP 22 38 2165

04-08-2022

| Patent document cited in search report | | Publication date | Patent family member(s) | | Publication date |
|---|---|---|---|---|---|
| WO 2020204457 | A1 | 08-10-2020 | KR | 20200114877 A | 07-10-2020 |
| | | | US | 2022213556 A1 | 07-07-2022 |
| | | | WO | 2020204457 A1 | 08-10-2020 |
| EP 1942197 | A1 | 09-07-2008 | AT | 382711 T | 15-01-2008 |
| | | | AU | 1218802 A | 13-03-2002 |
| | | | AU | 2002212188 B2 | 28-02-2008 |
| | | | CA | 2420840 A1 | 27-02-2003 |
| | | | DK | 1423528 T3 | 13-05-2008 |
| | | | EA | 200300330 A1 | 24-06-2004 |
| | | | EP | 1423528 A2 | 02-06-2004 |
| | | | EP | 1942197 A1 | 09-07-2008 |
| | | | ES | 2299520 T3 | 01-06-2008 |
| | | | IL | 154663 A | 24-12-2009 |
| | | | IS | 6705 A | 31-01-2003 |
| | | | JP | 2004516821 A | 10-06-2004 |
| | | | KR | 20040015705 A | 19-02-2004 |
| | | | MX | PA03001834 A | 01-08-2003 |
| | | | NZ | 524229 A | 27-01-2006 |
| | | | US | 2004234960 A1 | 25-11-2004 |
| | | | US | 2011136687 A1 | 09-06-2011 |
| | | | WO | 0218632 A2 | 07-03-2002 |

EPO FORM P0459

For more details about this annex : see Official Journal of the European Patent Office, No. 12/82

## REFERENCES CITED IN THE DESCRIPTION

**Patent documents cited in the description**

- WO 2008097543 A2
- WO 2016020551 A1
- US 20170022570 A1

**Non-patent literature cited in the description**

- Analytical performance of the ThyroSeq v3 genomic classifier for cancer diagnosis in thyroid nodules. **NIKIFOROVA MN. et al.** Cancer. John Wiley and Sons Inc, 2018, vol. 124, 1682-90
- **ALEXANDER EK. et al.** Preoperative Diagnosis of Benign Thyroid Nodules with Indeterminate Cytology. *New England Journal of Medicine,* 2012, vol. 367, 705-15
- **LABOURIER E. et al.** Molecular testing for miRNA, mRNA, and DNA on fine-needle aspiration improves the preoperative diagnosis of thyroid nodules with indeterminate cytology. *Journal of Clinical Endocrinology and Metabolism. Endocrine Society,* 2015, vol. 100, 2743-50
- DNA methylation in thyroid cancer. **ZAFON C. et al.** Endocrine-Related Cancer. BioScientifica Ltd, 2019, R415-39
- Comprehensive DNA Methylation Profiling Identifies Novel Diagnostic Biomarkers for Thyroid Cancer. **PARK JL. et al.** Thyroid. Mary Ann Liebert Inc, 2020, vol. 30, 192-203